# EUROPEAN PATENT APPLICATION

(11) **EP 1 006 182 A2**
(43) Date of publication of application: **07.06.2000**
(21) Application number: 00200860.5
(22) Date of filing: 11.01.1985
(51) Int. Cl.: C12N 15/12, C07K 14/755

(54) **Polypeptide fragments of factor VIIIC, corresponding probes and DNA sequences and DNA constructs**

(30) Priority: 12.01.1984 US 570062; 26.10.1984 US 664919
(62) Divisional of application: 91113267.8
(71) Applicant: CHIRON CORPORATION, Emeryville, California 94608 (US); NOVO NORDISK A/S, 2880 Bagsvaerd (DK)
(72) Inventor: Kuo, George, San Francisco, California 94122 (US); Rasmussen, Mirella Ezban, 2100 Copenhagen (DK); Masiarz, Frank R., San Francsco, California 94131 (US); Valenzuela, Pablo, San Francsco, California 94117 (US); Truett, Martha, Oakland, California 94611 (US); Favaloro, Jennifer, Carlton, Victoria 3035 (AU)
(74) Representative: Hallybone, Huw George

(57) **Abstract**

The present invention relates to a process for the synthesis of polypeptide fragments of human Factor VIIIC, particularly a fragment having a molecular weight of 77/80 kd, DNA sequences encoding portions of this polypeptide fragment, extrachromosomal elements comprising these DNA sequences, a complex probe for screening DNA sequences, corresponding DNA constructs, and monoclonal antibodies prepared with an immunogen comprising the above polypeptide fragments. The 77/80 kd fragment may be used for preparing a protein composition comprising a calcium bridged complex of the 77/80 kd polypeptide fragment with a 92.5 kd fragment, exhibiting a coagulation activity similar to that of human Factor VIIIC.

## Description

It is the object of the present invention to provide a process for the synthesis of polypeptide fragments of human Factor VIIIC, particularly a fragment having a molecular weight of 77/80 kd, DNA sequences encoding portions of this polypeptide fragment, extrachromosomal elements comprising these DNA fragments, a complex probe for screening DNA sequences, corresponding DNA constructs, and monoclonal antibodies prepared with an immunogen comprising the above polypeptide fragments.

The above object is achieved according to the claims. The dependent claims relate to preferred embodiments.

In accordance with the above-defined object, the present invention particularly relates to a process for the synthesis of polypeptide fragments of human Factor VIIIC, particularly a fragment having a molecular weight of 77/80 kd, DNA sequences encoding portions of this polypeptide fragment, extrachromosomal elements comprising these DNA sequences, a complex probe for screening DNA sequences, and corresponding DNA constructs.

The parent patent EP 150 735 (application number 85 100223.8-2105) relates to an isolated protein composition comprising a calcium bridged complex of the 77/80 kd polypeptide fragment with a 92.5 kd fragment, exhibiting a coagulation activity similar to that of human Factor VIIIC.

Factor VIIIC is a plasma protein that participates in the intrinsic pathway of blood coagulation. It is absent or defective in individuals with the hereditary X chromosome-linked recessive bleeding disorder hemophilia A. Great difficulty has been encountered in isolating Factor VIIIC due to its extremely low concentration in plasma and the fact that it appears to be an intermediate or final degradation product of a larger protein precursor. Therefore, efforts to isolate Factor VIIIC have led to complex mixtures of significant heterogeneity and varying molecular weights.

One of the approaches which has found broad application to the production of physiologically active proteins involves the isolation of the protein of interest in purified form. The protein of interest provides invaluable aid in the development of a recombinant DNA capability for the production of the protein. By having the protein of interest, one may prepare monoclonal antibodies which are specific for the protein and can be used to establish the production of the protein in lysates, expression from messenger RNA in oocytes, or from a cDNA gene in unicellular microorganisms. In addition, by amino acid sequencing, one can develop probes, employing codons coding for the particular amino acid sequence, for hybridization to messenger RNA, chromosomal DNA or cDNA and, therefore, provide for the detection, isolation and expression of the relevant gene or message and the production of the desired product in high yield in one or more hosts.

US-A-4 361 509 and references cited therein describe purification of Factor VIIIC (see also Fulcher and Zimmerman, Proc. Natl. Acad. Sci. USA (1982) 79:1648-1652). Tuddenham et al., J. of Lab. Clinical Medicine (1979) 93,40-53 describes purification of Factor VIIIC using polyclonal antibodies. Austen, British J. Hematology (1979) 43,669-674 describes the use of aminohexyl-Sepharose for Factor VIIIC purification. Weinstein et al., Proc. Natl. Acad. Sci. USA (1981) 78,5137-5141 describes a study of the effect of thrombin on Factor VIIIC. See also Kuo et al., Abstracts for IX International Congress of Thrombosis and Hemostasis, (Copenhagen; July, 1983).

It has further been known from C.A. Fulcher, J.R. Roberts and T.S. Zimmerman, Blood (1983) 61, No. 4, 807-811, that two polypeptide fragments of 79/80 kd and 92 kd can be isolated from the thrombin degradation product of human Factor VIIIC by SDS gel electrophoresis.

Methods and compositions are provided for production of human Factor VIIIC, precursors and subunits thereof, by expression in a microorganism or mammalian tissue culture cells. The method involves isolating pure Factor VIIIC, subunits and fragments thereof and determining their physiological relationship, particularly employing thrombin digestion. At least a portion of each of the related swiss of polypeptides is sequenced and the sequences employed for developing complex probes. Genomic DNA fragments are probed for homologous sequences and hybridizing fragments isolated and further manipulated to provide a DNA fragment encoding a complete subunit or fragment and/or used for isolating mature mRNA, from which ds cDNA may be obtained. The DNA sequence may then be further manipulated for insertion into an expression vector and the expression vector employed for introduction into a compatible host for expression of the polypeptide.

Human Factor VIIIC fragments and subunits are provided in substantially pure form. In addition, methods and compositions are provided for the expression of Factor VIIIC subunits and fragments for producing Factor VIIIC as a precursor or in its active form or providing individual subunits for use in combination with naturally available subunits. The subunits and fragments have one or more biological properties associated with Factor VIIIC, such as epitopic sites, coagulation activity, and immunogenicity, so as to be used for producing antibodies which find use as reagents, particularly labeled reagents in immunoassays.

Human Factor VIIIC is a complex protein which can be isolated in substantially pure form exhibiting an apparent molecular weight of about 460kd. Upon electrophoresis under denaturing conditions, a large number of fragments result of varying molecular weights: 240, 160, 140, 115, 92.5, 80 and 77kd, the latter two being found to migrate as a doublet. Analysis of the fragments by chemical and protease cleavage including thrombin, employing antibodies to follow immunogenic relationships and cleavage patterns to follow structural relationships, demonstrates that the 92.5kd polypeptide is related to the 240, 160, 140 and 115 polypeptides while the 77/80 doublet may not have common identifiable characteristics with the other peptides, except for the 240kd polypeptide. It is further found that the 77/80kd doublet is convened by thrombin to a 67/70kd doublet, while the 92.5kd polypeptide present in purified Factor VIIIC material treated with thrombin is cleaved by thrombin, directly or indirectly, into two polypeptides of about 40 and 52.5kd. It is found that the electrophoretically isolated 77/80kd doublet polypeptides have their N-termini blocked, while the 67/70kd doublet polypeptides do not have their N-termini blocked.

It is further found that the locus for Factor VIIIC involves exons with large introns, where exons involve various domains associated with Factor VIIIC. Thus, individual exons can be isolated which involve specific polypeptides or fragments thereof involved with the Factor VIIIC complex. By identifying specific amino acid sequences involved with Factor VIIIC subunits and fragments thereof, one can selectively isolate the exons from genomic DNA and use the exons by themselves, in combination, or joined by synthetic DNA to provide for sequences encoding for and production of polypeptide subunits of Factor VIIIC or fragments thereof.

Conveniently, the Factor VIIIC genomic DNA sequences containing both exons and introns may be inserted into an expression vector appropriate for transcription and translation in mammalian cells to provide for both substantial quantities of properly spliced messenger RNA suitable for cDNA cloning and production of Factor VIIIC, subunits or fragments. In addition, the DNA sequences isolated from the genome can be used for hybridizing to natural messenger RNA encoding for Factor VIIIC. The messenger RNA may then be used to prepare ds cDNA for encoding the subunits of Factor VIIIC. The DNA sequences may be employed for expression by insertion into an appropriate expression vector having the necessary regulatory signals for transcription and translation. The Factor VIIIC gene expression vector (an expression vector carrying one or more genes encoding for all or a portion of Factor VIIIC, precursor, subunits or fragments thereof) may be introduced into a compatible host and the host grown for expression of Factor VIIIC. By appropriate choice of hosts, the Factor VIIIC DNA may be inserted downstream from a secretory leader and processing signals, so that the product will be secreted from the host and processed to provide for the complete polypeptide. As appropriate, the polypeptide may be further processed to introduce functionalities or substituents present on the naturally occurring polypeptide.

In the first stage of the subject invention, highly purified Factor VIIIC is obtained and characterized. Purified Factor VIIIC can be obtained from commercially available human anti-hemophilic factor (AHF), which is prepared from fresh, normal human plasma as a cryoprecipitate and represents about a 40-fold enrichment. The Factor VIIIC is further concentrated and purified by dissolving the anti-hemophilic factor into an appropriate buffer, e.g., saline imidazole-lysine-HCl, pH 7.4, followed by chromatography on an affinity column having either polyclonal or monoclonal antibodies to Factor VIIIC or Factor VIIIR. Conveniently, the antibodies are covalently bonded to a Sepharose support. Factor VIIIC may be eluted from the column employing a combination of a relatively high concentration of calcium ion with glycerol. The fractions obtained from the column may then be dialysed with an appropriate buffer, as described above, containing a low concentration of calcium ion and may then be further purified employing an aminohexyl-Sepharose column eluted with a high calcium or sodium chloride concentration buffer. Additional chromatographic steps, e.g., gelatin Sepharose, HPLC, ion exchange on dextran sulfate or Mono Q, affinity columns using lectins or antibodies to Factor VIIIC, provide additional purification. Particularly, the use of dextran sulfate removes trace contamination, e.g., fibrinogen, fibronectin, IgG, from the preparation, so as to leave a product substantially free of foreign proteins. Activity of the fractions from the columns may be monitored for either or both biological and antigenic activity using coagulation assay (commercially available kits) and antibodies specific for Factor VIIIC. Based on the concentration of Factor VIII in plasma, purifications of about 200,000-fold may be achieved by the above-described method.

### Characterization of Factor VIIIC

Gel filtration indicates that Factor VIIIC behaves as a complex with an apparent molecular weight of about 460kd. Using SDS-gel electrophoresis (denaturing conditions) seven individual polypeptides can be isolated of differing molecular weight. The fragments as defined by their molecular weight are 240, 160, 140, 115, 92.5, 80 and 77kd. These fragments were characterized in the following ways.

The first study involved employing inhibitor antibodies isolated from hemophilic patients, the antibodies being designated as Z and E. Both antibodies reacted with the 77/80kd doublet. The E antibody reacted strongly with the 240kd polypeptide and weakly with several bands between the doublet and the 240kd polypeptide. The 2 antibody also reacted weakly with the 240kd polypeptide.

In immunoprecipitation experiments, the E antibody precipitates the 77/80kd doublet as well as the high molecular weight species of 160, 140, 115 and 92.5kd, with the doublet among the stronger bands. Inclusion of EGTA results in the loss of the bands other than the doublet indicating that the 92.5kd species is associated with the 77kd and/or 80kd species in a complex mediated by a Ca⁺⁺ bridge.

In the next study, monoclonal antibodies were prepared, which both inhibit Factor VIIIC mediated coagulation activity and react with components of the complex: Class I reacting with the 77/80kd doublet and 240kd polypeptides; Class II reacting with the 160, 140, 115 and 92.5kd polypeptides. Immunoprecipitation of thrombin-digested Factor VIIIC with Class I antibodies indicates that the 70/67kd doublet which results is derived from the 77/80kd doublet present in Factor VIIIC. The Class II monoclonals indicated that the 160, 140 and 115kd peptides are precursors of the 92.5kd peptide. A 40kd peptide cleavage product of 92.5kd peptide was also bound by the Class II antibodies. An ELISA assay using monoclonal antibodies in the presence and absence of EGTA confirms the Ca⁺⁺ bridge association between the 92.5kd and 77kd and/or 80kd components of the Factor VIIIC complex.

Both the human inhibitor and monoclonal antibodies may be used in immunosorbent column procedures to obtain Factor VIIIC or using EGTA, to resolve its constituent components, the 92.5kd and 77/80kd species.

The next study involved thrombin degradation of purified Factor VIIIC material at pH 6.8 or 7.4. Aliquots were assayed for coagulation activity and TCA precipitated for gel analysis. Coagulation activity was shown to increase with time and then decrease coincidently with an increase and decrease in the amount of the 92.5kd species. Thrombin treatment of the purified Factor VIIIC material for short periods of time (5-15min) enhances the amount of 92.5kd species, while the 77/80kd doublet is partially converted to a 67/70kd doublet. When long thrombin digestion times are employed, e.g., one hour, the 92.5kd protein is degraded and two new peptides of 40 and 52.5kd appear, with the 40kd peptide retaining immunogenic characteristics of the 92.5kd species. The 52.5kd peptide is shown to be a cleavage product by chemical and enzymatic degradation patterns and products analogous to the 92.5kd species.

In the next study individual Factor VIIIC subunits and precursors (e.g., 240, 77/80, 92.5kd species) were isolated by preparative SDS gel electrophoresis and a time course thrombin digestion of the isolated polypeptides was then performed. The 240kd fragment isolated from a preparative gel produced 160, 140, 115, and 92.5kd bands. The 80kd and 77kd fragments produced a 70kd and 67kd fragment, respectively.

A Factor VIIIC complex is derived containing the 77kd and/or 80kd species and 92.5kd polypeptide as a calcium-bridged complex in highly purified form. The purity of Factor VIIIC material (the complex and precursor species) is usually greater than 80%, often greater than 90%, and may be 98% or higher based on total protein, and that of the complex at least 20%, more usually 30% based on total protein, following the anti-Factor VIIIR immunosorbent and aminohexyl Sepharose columns. The use of additional chromatographic steps, e.g., dextran sulfate, increases the level of purity to at least 90% and usually greater for the Factor VIIIC material (complex plus precursor). The purity of Factor VIIIC components, 92.5kd species and the 77/80kd doublet, isolated by preparative SDS gel electrophoresis is usually at least 98%. As indicated above, the complex can be obtained using monoclonal antibodies specific for a member of the doublet, or for the 92.5kd polypeptide. The complex may then be separated from the antibody using a denaturing or chaiotropic solvent, e.g., aqueous urea or thiocyanate, respectively.

### Preparation of Probes

A partial amino acid sequence of the N-terminus of the 67 and 70kd polypeptides is as follows:

1 2 3 4 5 6 7 8 9 10 11
? Phe Gln Lys Lys Thr Arg His Tyr Phe Ile

12 13 14 15 16 17 18 19 20 21 22
Ala Ala Val Glu Arg Leu Trp Asp Tyr Gly Met

Based on this sequence, probes for the 67/70kd doublet (and thus the 77/80kd doublet from which it is derived) may be prepared having the following sequences:

Probe 1:  3' GTA ATA AAA TAA CGX CGX CA 5'          (X=G,C,A,T)
(non-coding)   G   G   G   G
                           T

Probe 2:  3' AAA GTT TTC TTC TGX TCT GT 5'
(non-coding)   G   C   T   T       C
                                  GCX

Probe 3:  5' GAA CGX TTA TGG GAT TAT GGX ATG 3'
(coding)       G AGA   G       C   C
                 G CTX

Probe 4:
(non-coding)
3' TCT GTA ATG AAA TAG CGA CGA CAC CTT TCT GAC ACC CTA ATG CCG TAC 5'
     C   G       G       G   G       C   C           G

The N-terminus amino acid sequence of the 52.5kd protein is substantially as follows:

1 2 3 4 5 6 7 8 9 10 11 12 13
Ala Thr Arg Arg Tyr Tyr Leu Gly Ala Val Glu Leu Ser

14 15 16 17 18 19 20 21 22 23 24 25 26
Trp Asp Tyr Met Gln Ser Asp ? Gly Glu Leu Pro Val

Based on this amino acid sequence, a probe for the 52.5kd protein may be prepared based on the coding strand as follows:

5'- GCA ACT AGA AGA TAT TAT TTG GGG GCA GTT GAA TTG TCA TGG GAT TAT -3'
          A   G   G           A   A       A       A   T

The amino acid sequences of three fragments of the 77/80kd proteins are as follows:

1 2 3 4 5 6 7 8 9 10 11 12
Tyr Ala Ala Thr Ser Gln Val Leu Leu Pro Ser Lys

1 2 3 4 5 6 7 8 9 10
Val Thr Gly Val Thr Thr Gln Gly Val Lys

1 2 3 4 5 6 7 8 9 10 11 12
Met Glu Val Leu Gly Cys Glu Ala Gln Asp Leu Tyr

Based on these sequences respectively, probes may be prepared based on the non-coding strands as follows:

Probe 1:  3'-ATA  CGT  CGT  TGA  AGT  GTT  CAA  AAC  AAC  GG-5'
                              T    A         T    T    T

Probe 2:  3'-CAA  TGA  CCT  CAA  TGA  TGA  GTT  CCT  CAA  TTT-5'
               T    T         T    T    T              T

Probe 3:  3'-TAC  CTT  CAA  AAC  CCT  ACG  CTT  CGT  GTT  CTA  AAC  ATA-5'
                         T    T                                  T

Sequences of these peptides and preparation of the corresponding probes are described in detail in the Experimental section hereinafter.

### Isolation of DNA

The above probes can be used for the detection and isolation of either genomic DNA or messenger RNA. Cloning genomic DNA involves cleavage of the genomic DNA with one or more restriction enzymes to obtain a partial digest and size selection of fragments of about 10-25kb. The restriction digests should be incomplete so that there will be overlapping fragments cloned. These fragments may be cloned in the appropriate vector to produce a "library" of clones in microorganisms, e.g., bacteria, such as E. coli. Various vectors may be employed, including plasmids or viruses, such as pBR322, lambda, charon 4A, EMBL-4, or the like.

The DNA is screened with the enzymatically radiolabeled probes described above and homologous sequences detected. Those sequences which hybridize with one or more of the probes may be recloned and again rehybridized one or more times.

One or more restriction enzymes different from the original restriction enzyme(s) employed may then be used to provide for smaller fragments, generally ranging from about 1-10kb, more usually from about 1-6kb. These fragments may then be subcloned and screened to identify positive fragments. The synthetic probes can then be used as primers for sequencing of the DNA fragments. Fragments which may be most conveniently sequenced are those which include a sequence complementary to one or more of the above-identified probes, where the homologous sequence is from about 5 bases and up to not more than about 500 bases from the 5'-terminus. Other fragments of interest include those at the termini of the original cloned fragment since these will be represented in other clones in the library and thus used to "walk" along the chromosome until the entire desired gene is recovered.

After sequencing the DNA fragment, based on the determined sequence, the fragment will be further manipulated. Based on the sequence, one can identify an open reading frame including the determined amino acid sequence. By determining restriction sites, one can further reduce the size of the fragment, without loss of coding sequences, although removal of a short sequence at the N-terminus is permissible, since this can be replaced by using appropriate adapters. Where restriction sites are not readily available at appropriate positions, the DNA fragment may be modified by Bal31 resection for varying times, the resulting fragments cloned, and the 5'-termini determined by various techniques. Conveniently, one can provide for a recognition site of a particular restriction enzyme by appropriate selection of the 3'-bases to which the resected fragment is joined. In this way, one can screen the resulting clones for the predetermined restriction site, which will indicate the presence of a fragment resected to the desired site.

Desirably exons or fragments thereof, usually of at least 50bp, more usually of at least about 100bp, even about 250bp or more, may be denatured and used as probes for mRNA from human cells, particularly cells producing mRNA for Factor VIIIC. By isolating hybridizing mRNA, the mRNA may be screened by translation in oocytes or a reticulocyte lysate and production of Factor VIIIC detected by antibodies to Factor VIIIC or coagulation activity based on binding to Factor VIIIC subunits. The mRNA may then be reverse transcribed, using, for example, AMV reverse transcriptase. Various methods can be used for converting ss cDNA to ds cDNA, using the reverse transcriptase or DNA polymerase I (Klenow fragment) to produce the second strand, followed by removal of the terminal loop, as appropriate, with a nuclease, e.g., S₁ nuclease. Where an incomplete copy is obtained, the messenger may be "walked" or primed cDNA synthesis may be used until the 5'-coding sequence of the mRNA has been copied and a DNA sequence encoding for the entire coding region of the mRNA is obtained.

Based on the above procedures, DNA sequences coding for the polypeptide precursor(s) to Factor VIIIC or major fragments thereof may be used for expression, or smaller fragments coding for specific subunits of Factor VIIIC, e.g., 92.5kd, 80kd or 77kd, may be employed.

For the precursor polypeptide, (proFactor VIIIC), the gene may be blunt-ended at one or both ends and inserted into an expression vector having complementary ends, or may be cleaved downstream from the 5'-coding terminus and joined to an adapter for appropriate insertion into the vector.

Fragments having the proper N-terminus, which may be at the coding sequence for the 70kd or 80kd polypeptide or may have a 5'-terminus downstream from the initial base, usually not more than about 30 bases downstream, more usually not more than about 20 bases downstream, may then be inserted into an appropriate vector using adapters, as appropriate.

Various vectors may be employed for providing extrachromosomal elements, depending upon the particular host, the manner of expression, whether constitutive or induced, the desired markers, whether secretion is desired, or the like. (By vector is intended an intact replication system.) Numerous vectors are presently available which provide for the transcriptional and translational regulatory signals recognized either by mammalian hosts, e.g., tissue culture cells or by prokaryotic and eukaryotic microorganism hosts, e.g., E. coli, B. subtilis, B. thermophilus, S. cerevisiae, or the like.

The vectors will hive a replication system recognized by the host, although in some instances, integration of a construct having transcriptional and translational regulatory signals and the cistron of interest into the host genome may be desirable. In those situations, the construct will usually be flanked by sequences homologous to sequences in the host genome.

The expression vectors which are employed will have transcriptional and translational signals recognized by the host. The transcriptional signals will include the promoter and terminator, as well as auxiliary signals such as one or more enhancers. In addition, regulation of transcription may be provided, by including operators, activators, genes providing for repression, or the like. Other sequences involved with transcription include capping, polyadenylation, etc. For translation, depending upon the host, there may be a ribosomal binding site, an initiation codon, stop codons, or the like.

Conveniently, non-coding 5'- and 3'-flanking regions will be employed from genes native to the host, so that the signals recognized by the host will be present in appropriate relationship. These flanking regions can be joined to the gene encoding for the Factor VIIIC precursor, subunit or fragment thereof, so that the gene is in reading frame with the initiation codon and either carries its own stop codon or is inserted immediately upstream from one or more stop codons.

A vector will normally have one or more markers which provide for selection and allow for continued selective pressure during growth of the host. These markers may include prototrophy in an auxotrophic host, antibiotic resistance, toxin resistance, etc.

Where a secretory leader and processing signals are provided, it will usually be necessary to provide an adapter. By providing for an appropriate restriction site at the terminus of the DNA sequence encoding secretory leader and processing signals or upstream therefrom, one can synthesize an oligonucleotide adapter, usually of from about 10-50bp, which can be inserted between the secretory leader and processing signals or truncated portion thereof, and the gene of interest, which has a 5'-terminus at the initial codon of the gene or downstream thereof, so that the adapter restores all of the necessary missing bases and provides for the gene being in reading frame with the initiation codon of the leader sequence.

The resulting constructs which include the desired gene may then be introduced into a host, capable of growth in culture, in accordance with conventional methods, e.g., transformation, conjugation, transfection, or the like. The host may then be grown in an appropriate nutrient medium and the product isolated in accordance with conventional ways. Where the product is retained intracellularly, the cells will be harvested and lysed: where secreted, the product will be isolated from the nutrient medium. The product may be purified by chromatography, e.g., affinity chromatography, electrophoresis, extraction, HPLC, or the like.

For expression in a mammalian cell a mammalian virus may be employed as the vector, e.g., SV-40, papilloma virus, Maloney murine sarcoma virus, adenovirus, or the like. These viruses have been modified for use as expression vectors in mammalian cell cultures. An illustrative system employs COS cells bearing an integrated SV-40 genome and producing the large T antigen required for SV-40 replication (Gluzman, Cell (1981) 23:175). A fragment spanning the HpaI site at 0.76 on the SV-40 map to the BamHI site at 0.14 on the SV-40 map may be used as a vector. The recombinant plasmid obtained by joining the Factor VIIIC gene or portion thereof with the SV-40 vector may be used to transfect monkey CV-1 cells.

In accordance with the subject invention, purified subunits and fragments of Factor VIIIC may be obtained and used to enhance clotting capability for individuals requiring the particular subunit. The Factor VIIIC may also be used in therapy. In addition, the polypeptides prepared according to this invention can be used tor the production of monoclonal antibodies to Factor VIIIC, its subunits and fragments. Also, the subunits and fragments may be used as reagents, which may be labeled and in combination with the antibodies, employed in diagnostic assays for the presence of one or more subunits or degradation fragments thereof in physiological fluids, e.g., blood or serum.

The following examples are offered by way of illustration and not by way of limitation.

Whenever used hereinafter Ab intends antibody and Ag antigen.

### EXPERIMENTAL

### I. Purification of Factor VIIIC

Human Factor VIIIC was isolated from commercial cryoprecipitate preparations by a) immunosorbent chromatography using a polyclonal anti VIIIR-Sepharose column by a method first described by E.G.D. Tuddenham, N.C. Trabold, J.A. Collins, and L.W. Hoyer, J. of Lab. Clinical Medicine (1979) 93:40; and b) a chromatographic separation on aminohexyl-substituted agarose as was originally described by D.E.G. Austen, British J. of Hematology (1979) 43:669.

Details of the procedures are described below.

Goat anti-human Factor VIII Related Antigen (VIII:R) serum obtained from Atlantic Antibody (cat. no. 040-01), was treated with either a standard 0-50% ammonium sulfate cut followed by DEAE cellulose column chromatography, or a similar 0-33% cut without subsequent chromatography. These materials were then conjugated to CNBr-activated Sepharose CL2B or 4B, respectively, (Pharmacia, 17-0140-01 or 17-0430-01) and poured as a column (anti VIII:R-Sepharose column).

"HEMOFIL", a stable, dried preparation of antihemophilic factor (Factor VIII, AHF, AHG) in concentrated form prepared from fresh, normal human plasma, representing about a 40-fold enrichment for Factor VIIIC, was dissolved in the following buffer: 0.02M imidazole, 0.15M NaCl, 0.1M lysine-HCl, 0.02% NaN₃, pH 7.4.

After being dissolved, the Hemofil was applied to the above-described anti VIII:R-Sepharose column. Non-specifically bound protein was eluted with the above buffer modified to 0.5M NaCl. Next, Factor VIIIC was eluted with the above buffer containing 0.35M CaCl₂, with the addition of 10% glycerol which stabilizes the Factor VIIIC activity. Active fractions from the immunosorbent column were pooled and dialyzed against buffer (0.02M imidazole, 0.15M NaCl, 0.1M lysine-HCl, 0.025M CaCl₂, 0.02% NaN₃, 10% glycerol, pH 7.4). An aliquot of the dialyzed fractions, which contained 1,100 units of Factor VIIIC, was applied to an aminohexyl-Sepharose 4B column (1x6cm) equilibrated with dialysis buffer described above. Factor VIIIC activity was eluted with the same buffer containing either 0.35M CaCl₂ or 2M NaCl. The activity was found to be in a volume of 2ml with 500 units of Factor VIIIC per ml. Subsequent experiments carried out in the same manner provided a recovery of 25% off the anti VIII:R column and a recovery of approximately 90% off the aminohexyl column. Alternatively, pooled, dialysed material eluted from the immunosorbent column is first applied to a dextran sulfate (Pharmacia) column (1.5 x 6cm) equilibrated with the dialysis buffer above and eluted with the same buffer. Several minor contaminants, e.g., fibrinogen, fibronectin, IgG, are retained on the column while Factor VIIIC emerges in the flow-through which is collected and loaded on the aminohexyl-Sepharose column as before.

Both biological, i.e., cloning, and antigenic (cAg) activity were shown to be present in the purified Factor VIIIC, as demonstrated by the subsequent assays indicating a 5,000-fold purification over the 40-fold concentration in Hemofil. Using a standard commercially available three component kit from General Diagnostics, (APTT, Factor VIII deficient plasma, Verify Normal Citrate) a coagulation assay was carried out and indicated high levels of Factor VIIIC biological activity.

Antibodies employed were derived from inhibitor patients, one with a low titer (LZ) as coating ab and one with a high titer (HZ) as the labeled ab. The antibodies were used in two different types of assays. In an RIA assay, the HZ ab is labeled with I¹²⁵, while in an ELISA assay the HZ ab is coupled to horseradish peroxidase. Labeling with ¹²⁵I of antibody HZ for the RIA was performed in accordance with Hunter, W.M., In Radioimmunoassay, Weir, D.M., ed., Handbook of Experimental Immunology, 3rd ed., vol. 1, Blackwell Scientific Publications, Oxford, 1978. HRP-HZ conjugation was in accordance with Wilson and Nakane, In Immunofluorescence and Related Staining Techniques, Knapp et al., eds., Elsevier, North-Holland Biomedical Press, Amsterdam, 1978, pp. 215-224. LZ had an activity of 700 Bethesda Units/ml while HZ had an activity of 1,500 Bethesda Units/ml. Coating antibody (LZ) was diluted to 3.5µg/ml in 0.1M NaHCO₃, pH 9.8 (RIA) or 0.05M imidazole, 0.1M NaCl, 0.01% Thimerosal, 0.05% Tween 20, 5% BSA (ELISA) or for either method PBS-CMF (for 1 liter: 200mg KCl, 200mg KH₂PO₄, 8.0g NaCl, 1.15g anhydrous Na₂HPO₄, pH 7.4) and 1 ml added to each tube (polystyrene) and incubated overnight at room temperature. This solution is removed by suction and the tubes washed 3x with 3-3.5ml 0.15M NaCl or PBS-CMF containing 0.05% Tween 20. Samples or standards (General Diagnostics, Verifiy Normal Citrate, catalog #34112) are diluted and added to the tubes to a total volume of 0.9ml per tube and incubated overnight at room temperature (dilutions were made in 0.02M Tris, 0.15M NaCl, 5% BSA, 0.05% Tween 20, 0.01% Thimerosal, pH 6.5 for RIA or 0.05M imidazole, 0.1M NaCl, 0.01% Thimerosal, 0.05% Tween 20, 5% BSA for ELISA or PBS-CMF for either method). Solutions were removed by suction and tubes washed as before. For RIA, 5 x 10⁵ cpm of ¹²⁵I-labeled antibody to Factor VIIIC (HZ) in 600µl of RIA dilution buffer was added to each tube which was then incubated at 37° C for 16-18h; solutions were removed, the tubes washed as before and counted in a gamma counter. For ELISA, 0.9ml peroxidase conjugated anti-Factor VIIIC (HZ) was added to each tube whch was then incubated overnight at room temperature; solutions were removed and the tubes washed as before, then 0.9ml OPD solution (for 100ml: 0.73g citric acid, 1.19g disodium acid phosphate, 0.15g o-phenylenediamine, pH 5.0 with 250µl 10% K₂O₂ added immediately before use) added and incubated at room temperature for 30min in the dark. To stop this reaction, 0.5ml at 6N HCl (or 0.9ml 1M H₂SO₄) was added to each tube and the OD₄₉₂ read.

### II. Structure of the Factor VIIIC Complex

### A. Immunoprecipitation Experiments

Gel filtration experiments were carried out with an AcA 44 column on the Factor VIIIC purified material under the following conditions: 0.1% insulin (as carrier protein for stabilization), 0.25M CaCl₂, 0.01% Thiomerosal, 0.05M imidazole, pH 7.2. The Factor VIIIC coagulation and antigenic activities of the eluate were monitored. Two antigenic peaks were observed. One with Factor VIIIC coagulation activity behaved as a complex with an apparent molecular weight of about 460,000 under these conditions (native). The other peak (devoid of coagulation activity) eluted at an observed molecular weight slightly below 67,000.

When analyzed by standard analytical Laemmli SDS-gel electrophoresis (Laemmli, Nature (1970) 227:680-685), various protein species of 240, 160, 140, 115, 92.5, 80 and 77kd were obtained. The relationship of these proteins to Factor VIIIC was determined by standard immunoprecipitation procedures. In the immunoprecipitation procedure, S. aureus protein A-Sepharose CL4B or polystyrene beads (1/8in, Precision Plastic Ball Co.) coated with affinity purified second antibody (goat anti-mouse IgG or anti-human IgG) were employed to separate antigen-Ab complexes from free ¹²⁵I-labeled Factor VIIIC.

The proteins eluted from the affinity column were iodinated and then reacted with antibodies specific for Factor VIIIC. The antibodies were human inhibitor antibodies isolated from hemophiliac patients and referred to as anti-Factor VIIIC (Z) and (E) or inhibitor antibody (Z) and (E).

The results indicated that both antibodies reacted with the 77/80kd doublet The "E" antibody also reacted strongly with the 240kd band and gave weak precipitation of several bands (160, 140, 115, 92.5kd) between the doublet and 240kd species. The "Z" antibody also precipitated the 92.5kd and 240kd proteins. The strong reaction of the "E" antibody with the 240kd species suggests that this species is a precursor of Factor VIIIC.

The antibody-column purified Factor VIIIC fraction was iodinated and reacted with the human inhibitor antibody in the presence and absence of EGTA (ethylene glycol bis(β-aminoethyl ether) N,N,N',N'-tetracetic acid). This allows for an investigation of the role of divalent cations, particularly Ca⁺⁺, in the association of the Factor VIIIC polypeptides. It was observed that the inhibitor antibody (E) precipitates the 77/80kd doublet, as well as higher molecular weight species of 160, 140, 115 and 92.5kd. The doublet is always among the stronger bands. (This immunoprecipitation experiment was done with the polystyrene beads. This procedure results in lower backgrounds and the labeled IgG in the Factor VIIIC preparation is not precipitated). Inclusion of EGTA results in the loss of the higher molecular weight bands (92.5-160kd) but has no effect on the amount of doublet precipitated. A similar experiment utilized Z antibody coupled to Sepharose as an immunosorbent: purified Factor VIIIC is applied to the column and after binding via 77/80kd, the 92.5kd polypeptide is selectively eluted with EDTA (ethylene diamine tetraacetic acid). The method is used preparatively to fractionate the 92.5kd species. This immunosorbent column or a similar one are prepared with polyclonal antibodies to Factor VIIIC. When eluted with chaiotropic or denaturing solvents, e.g., thiocyanate solutions or aqueous urea, respectively, rather than EGTA, Factor VIIIC is further purified. These results suggest that the 92.5kd peptide may be associated noncovalently to the 77/80kd doublet via a Ca⁺⁺ bridge. Inhibitor antibody appears to interact directly only with the doublet. The higher molecular weight bands (the 115kd, 140kd, 160kd) are probably precursors of 92.5kd, as indicated by the ability of the monoclonal antibody directed against the 92.5kd polypeptide to cross-react with the 115kd, 140kd and 160kd polypeptides.

The relationship of various protein species from the affinity column was demonstrated by immunoprecipitation of iodinated, purified Factor VIIIC with monoclonal antibodies prepared according to the method of G. Kohler and C. Milstein (Eur. J. of Immunol. (1975) 6:511). Balb/c mice were immunized with liquid phase immunoadsorbed Factor VIIIC. Spleen cells (10⁸) were fused with 10⁷ NSO or NSI mouse myeloma cells. The fusion products were plated into two 96-well microtiter trays. A spleen cell feeder layer was used at 10⁴ cells/well. Colonies were microscopically visible from the fifth day and the supernatants assayed every few days using an ELISA assay. The following layers were employed: 1st, Factor VIIIC eluted from hexyl-Sepharose 4B column, as described in Section I above; 2nd, hybridoma cell supernatant; 3rd, horseradish peroxidase (HRP)-labeled goat anti-mouse IgG; 4th, HRP-substrate.

Several classes of monoclonal antibodies were identified, two of which inhibited Factor VIIIC coagulation activity: Class I antibodies reacted with the 80/77kd doublet and 240kd polypeptides; and Class II antibodies reacted with proteins of 240, 160, 140, 115, 92.5kd. Immunoprecipitation of thrombin-digested Factor VIII with Class I monoclonal antibodies indicates that the 70/67kd doublet produced is derived from 77/80kd doublet (see below). Class III monoclonal antibodies indicate that the 160, 140 and 115kd peptides are precursors of 92.5kd peptide. The monoclonal antibodies of Class III further reacted with a 40kd peptide produced by thrombin digestion of the purified Factor VIIIC material.

An experiment similar to that described above, using EGTA to investigate the role of Ca⁺⁺ ion in the Factor VIIIC complex, was also performed utilizing a monoclonal antibody based ELISA assay with the following layers: 1st, monospecific anti(mouse IgG); 2nd, Class III monoclonal antibody (anti-92.5kd); 3rd, purified Factor VIIIC material; 4th, HRP-human inhibitor antibody to 77/80kd. Addition of EGTA removed bound HRP activity present in the control without chelator. The fact that the Class I and III monoclonal antibodies directed to the 77/80kd doublet and 92.5kd proteins, respectively, are each inhibitory to Factor VIIIC coagulation activity implicates both as essential components of the Factor VIIIC complex.

### B. Thrombin Activation of Factor VIIIC

Arninohexyl-concentrated, affinity-purified Factor VIIIC has been activated by thrombin (Boehringer, lot #1072302) using two different sets of pH conditions (6.8 and 7.4).

Aliquots were assayed for coagulation activity and, in addition, samples (about 2.5 units each) were TCA precipitated for gel analysis. In the first experiments, the VIIIC activity was initially 46 units/ml. This was diluted to a final concentration of 11.5 units/ml in Factor VIIIC buffer (20mM imidazole, pH 6.8, 150mM NaCl, 100mM lysine, 25mM CaCl₂ and 10% glycerol). The final concentration of thrombin was 0.12 unit/ml (about 1 unit of thrombin per 100 coagulation units of VIIIC). The results showed that the coagulation activity increases to about 180 units/ml then decreases to about 40 units/ml (essentially the starting value) coincidentally with a similar increase and decrease in the amount of 92.5kd species. Thus the 92.5kd species is implicated as part of the active Factor VIIIC complex.

Additional experiments with more concentrated Factor VIIIC preparations were carried out for the purpose of using thrombin activation in a preparative manner. To generate 92.5kd polypeptide, thrombin was added to the purified Factor VIIIC material (pH 7.4) at a ratio of about 1000-2000 coagulation units of Factor VIIIC to 1 unit of thrombin activity and allowed to react for only a short period of time (5-15min, depending on the Hemofil sample). The resulting product was then applied to a 7.5% preparative gel and peptides separated by electrophoresis, the gel bands cut out and electroeluted.

When thrombin digestion is carried out for a short time, the amount of 92.5kd species can be doubled or tripled; at the same time, the 77/80kd doublet is only partially convened to 67/70kd species. To optimize conditions for isolation of the 67/70kd doublet, a longer time course (greater than 1h) thrombin digestion is carried out. In this case, the 92.5kd species is further cleaved to produce smaller fragments. Two new peptides, 52.5kd and 40kd appear after thrombin treatment. The 40kd peptide reacts with the monoclonal antibody directed against the 92.5kd species and must therefore be a cleavage product. The 52.5kd peptide is also derived from the 92.5kd protein as demonstrated by a comparison of chemical and enzymatic cleavage patterns, i.e., both the 92.5kd and 52.5kd species when subjected to CNBr or endoproteinase lys C cleavage show a number of common fragments (by SDS-PAGE).

For endoproteinase lys C digestion, a weight ratio of lys C to protein of from about 1:1-100, usually 1:10, is used. In the subject digestion, 20pmoles (4.8µg) lys C was combined with 200pmoles (14µg) 70kd polypeptide in about 100µl 0.025M Tris-HCl, pH 7.7, 0.001M EDTA, 0.08% SDS and the mixture incubated at 37° C for 6h to overnight for complete digestion. Native polyacrylamide gels according to Orstein, Ann. N.Y. Acad. Sci. (1964) 121:321-349 were used for isolation of lys C digestion products.

### C. Thrombin Digestion of Gel Isolated VIIIC-related Proteins

In order to confirm the precursor-product relationship of these peptides, a number of the bands were isolated by preparative SDS gel electrophoresis, electroeluted and subjected to thrombin digestion. The results were as follows:
1. The 240kd protein produced multiple bands including 160, 140, 115, 92.5kd but nothing smaller than 92.5kd, i.e., no 77/80kd or 67/70kd doublet. In addition, a time course for digestion was carried out with the 240kd fragment and analyzed for gel electrophoresis pattern, coagulation activity, and Factor VIIIC antigenic (Cag) activity. Gel results were the same as above and essentially no Cag or coagulation activity was recovered.
2. The 160kd and 92.5kd gel-isolated polypeptides do not appear to be substrates for thrombin after isolation from the gel.
3. Thrombin specifically cleaves gel isolated 77kd and 80kd species to produce new polypeptides of 67kd and 70kd, respectively. After thrombin treatment, monoclonal antibodies of Class I precipitate not only the 77/80kd doublet, but also the new 67 and 70kd species.

### D. Amino Acid Sequence Analysis

Partial amino acid sequence information was obtained by standard procedures for the 67/70kd peptides, the 77/80kd peptides and the 52.5kd peptide isolated by preparative SDS electrophoresis. The electrophoretic analysis, together with the amino acid sequence results, indicated that the gel-isolated 77/80kd, 67/70kd, 92.5kd and 52.5kd polypeptides were obtained at >95%, usually 98%, purity. The gel isolated peptides were applied to a gas phase protein sequencer (Applied Biosystems). The PTH-amino acids were applied to an HPLC column (IBM cyano, 25cm) and the amino sequence determined from the resulting chromatograms.

The following sequence was determined for the 67/70kd doublet at its amino terminus (indicated with a bar in Appendix B): 
1 2 3 4 5 6 7 8 9 10 11
? Phe Gln Lys Lys Thr Arg His Tyr Phe Ile

12 13 14 15 16 17 18 19 20 21 22
Ala Ala Val Glu Arg Leu Trp Asp Tyr Gly Met
 Using the information provided by the amino acid sequence of the N-terminal region of the 67/70kd protein the following oligonucleotide probes were synthesized to be used to screen human genomic libraries. The phosphoramidite method as described by M.S. Urdea et al., Proc. Natl. Acad. Sci. USA (1983) 80:7461-7465 was used:

Probe 1: 3' GTA ATA AAA TAA CGX CGX CA 5' (X=G,C,A,T)
G G G G
T

Probe 2: 3' AAA GTT TTC TTC TGX TCT GT 5'
G C T T C
GCX

Probe 3: 5' GAA CGX TTA TGG GAT TAT GGX ATG 3'
G AGA G C C
G CTX

Probe 4:
3' TCT GTA ATG AAA TAG CGA CGA CAC CTT TCT GAC ACC CTA ATG CCG TAC 5'
C G G G G C C G

A scheme showing regions of amino acid sequence from which each probe is derived is shown below:

1 2 3 4 5 6 7 8 9 10 11 12 13 14 15 16 17 18 19 20 21 22
? Phe Gln Lys Lys Thr Arg His Tyr Phe Ile Ala Ala Val Glu Arg Leu Trp Asp Tyr Gly Met

AAA GTT TTC TTC TGX TCT GTA ATA AAA TAA CGX CGX CA GAA CGX TTA TGG GAT TAT GGX ATG
G C T T GCC G G G G G AGA G C C
G T G CTX
A

PROBE 1-20mer (384) PROBE 3-24mer (1152)
PROBE 2-20mer (512)
30-60% GC 38-63% GC
20-55% GC
Tm=52-64°C (Tm=66-78°C)
Tm=48-62°C
PROBE 4-48mer (256)

For the 52.5kd protein, which, as shown below, is derived from the 92.5kd protein) the following amino acid sequence at the N-terminus was determined:

1 2 3 4 5 6 7 8 9 10 11 12 13
Ala Thr Arg Arg Tyr Tyr Leu Gly Ala Val Glu Leu Ser

14 15 16 17 18 19 20 21 22 23 24 25 26
Trp Asp Tyr Met Gln Ser Asp ? Gly Glu Leu Pro Val

Based on the amino acid sequence for the 52.5kd peptide, a partially degenerate probe having the following nucleotide sequence (coding) was synthesized: 
5'-GCA  ACT  AGA  AGA  TAT  TAT  TTG  GGG
          A    G    G              A    A

   GCA  GTT  GAA  TTG  TCA  TGG  GAT  TAT-3'
          A         A    T
 This probe is useful for screening both genomic and cDNA libraries.

The amino acid sequences of two peptides obtained by digestion of the 77-80kd doublet with endoproteinase LysC (Boehringer-Mannheim) were determined. The digestion was performed as follows. The 77-80kd doublet was electrophoresed on an acrylamide protein gel and bands corresponding to the doublet were electroeluted. The separated material was purified and digested with endoproteinase LsyC, and the resulting peptides were separated by reverse phase HPLC. The fractions corresponding to peaks of absorbance at 280nm were sequenced using an automated sequencer (Applied Biosystems, Foster City, CA, Model A70A). The first sequence was as follows:

1 2 3 4 5 6 7 8 9 10 11 12
Tyr Ala Ala Thr Ser Gln Val Leu Leu Pro Ser Lys

Based on this amino acid sequence, a partially degenerate probe having the following nucleotide sequence (non-coding) strand was synthesized:

3'-ATA  CGT  CGT  TGA  AGT  GTT  CAA  AAC  AAC  GG-5'
                   T    A         T    T    T

The second peptide had the following sequence:

1 2 3 4 5 6 7 8 9 10
Val Thr Gly Val Thr Thr Gln Gly Val Lys

Based on this amino acid sequence, a partially degenerate probe having the following nucleotide sequence (non-coding) was prepared:

3'-CAA  TGA  CCT  CAA  TGA
     T    T         T    T

   TGA  GTT  CCT  CAA  TTT-5'
    T              T

The 77/80kd doublet was also digested with trypsin. The doublet material was purified as described for digestion with endoproteinase LysC, above, Lysines were blocked by citraconylation to allow digestion only at arginines. Citraconylation was performed by suspending the proteins in a denaturing buffer, reducing and carboxymethylating the suspended proteins, and treating with citraconic anhydride while maintaining a pH between 8.5 and 9.0. After citraconylation, the proteins were digested with trypsin, and the resulting peptides separated by reverse phase HPLC. The fractions corresponding to peaks of absorbance at 280nm were sequenced, as above. The sequence was as follows:

1 2 3 4 5 6 7 8 9 10 11 12
Met Gly Val Leu Gly Cys Glu Ala Gln Asp Leu Tyr

Based on this amino acid sequence, a partially degenerate probe having the following nucleotide sequence (non-coding) was synthesized:

3'-TAC CTT CAA AAC CCT ACG CTT CGT GTT CTA AAC ATA-5'
            T   T                           T

When procedures to determine the N-terminus sequences of the 80 and 77Kd species were carried out, it was found that their amino termini were blocked. Therefore, the N-termini sequence was determined from material obtained by an alternative purification method which included immunoaffinity chromatography and ion exchange chromatography and precluded the use of preparative SDS-polyacrylamide gel electrophoresis. Purification of the 80/77Kd doublet involved application of Factor VIIIC concentrate to a monoclonal antibody column followed by chromatography on a mono S cation exchanger. This material had an unblocked N-termini, indicating that the blockage detected in gel purified 80/77Kd was an artifact resulting from the gel electrophoresis. The amino termini sequences determined for both 80 and 77Kd species is the following (indicated with a bar in Appendix B):

1 2 3 4 5 6 7 8 9 10
Glu Ile Thr ? ? ? Leu Gln ? Asp
11 12 13 14 15 16 17 18 19 20
Gln Glu Glu Ile Asp Tyr ? Asp Asp Ile

### E. Amino Acid Composition

The amino acid compositions for the 77/80kd peptides were determined by standard methods to be as follows:

| Amino Acid | 80K | 77K |
|---|---|---|
| Asp | 58 | 54 |
| Glu | 74 | 76 |
| Cys | 12 | 14 |
| Ser | 47 | 44 |
| Gly | 51 | 46 |
| His | 8 | 12 |
| Arg | 32 | 29 |
| Thr | 35 | 29 |
| Ala | 35 | 33 |
| Pro | 33 | 30 |
| Tyr | 25 | 25 |
| Val | 46 | 44 |
| Met | 17 | 17 |
| Ile | 33 | 35 |
| Leu | 49 | 48 |
| Phe | 32 | 31 |
| Lys | 47 | 41 |
| Total No. Amino Acids | 634 | 608 |
| Calculated Molecular Weight: | 82K | 79K |

### F. Preparation of Human 4X Genomic Library

Approximately 3mg of DNA were prepared from cell culture lysates of GM1416 cells (human lymphoblastoid cell line containing 4 copies of the X chromosome).

This DNA was partially digested with the restriction enzyme Sau3A, and the digested DNA (400-500µg) fractionated on 10%-40% sucrose gradients. Fractions in the size range 10-25 kilobases were pooled, dialyzed into Tris-EDTA and purified over Schleicher and Schuell Elutip-d sterile disposable columns. Aliquots of this DNA were ligated to EMBL-4 arms, obtained after digestion with BamHI and SalI and isolation on a gradient, and then packaged into bacteriophage lambda with an efficiency of 1x10⁶ pfu/µg of insert DNA. The vector used, EMBL-4, is a modified form of bacteriophage lambda (see Karn et al., Methods Enzymol. (1983) 101:3-19). The total library consisted of 5x10⁶ phage.

### G. Plating and Screening of Human 4X Genomic Library

Bacteriophage were adsorbed to E. coli strain DP50 and 20 plates were plated at 50,000pfu per plate, (150x15mm size) to give 1x10⁶pfu total. (Details of techniques for plates, top agar, adsorption and plating are found in Molecular Cloning, A Laboratory Manual, by T. Maniatis, E.F. Fritsch and J. Sambrook; Cold Spring Harbor Lab, New York, 1982.)

Nitrocellulose filters were applied to the surface of each plate containing phage plaques (so that molecules of unpackaged phage DNA are transferred to the filters) in duplicate, and hybridized with ³²P-labeled 256-fold degenerate 48-mer probe DNA (probe #4). (Details of the nitrocellulose transfer technique are found in Maniatis et al., supra.)
Pre-hybridization and hybridization were carried out in Wallace mix which contains in one liter: 310ml of distilled H₂O, 200ml 50% dextran sulfate, 180ml 1M Tris-HCl, pH 8.0, 225ml 4M NaCl, 20ml 0.25M EDTA, 50ml 100X Denhardt's solution, 5ml 100% NP-40 and 10ml 10% SDS.

The probe was labeled by enzymatic transfer of ³²PO₄ from γ-ATP³² to the 5' phosphate end of each probe DNA molecule, catalysed by T4 polynucleotide kinase. The hybridization conditions were as follows: 10ml hybridization mix/filter x 5000cpm of labeled probe #4/degeneracy/ml. Hybridization was carried out at 37° C overnight. Filters were washed in 6XSSC, 1mM EDTA at 50-55° C, air dried and used to expose X-ray film.

### H. Characterization of Positive Clones

Twenty-three plaques giving positive signals for the first round of screening were replated, phage DNA transferred to nitrocellulose and hybridized with freshly labeled probe #4 (secondaries). Eleven plaques giving positive signals were replated. phage DNA transferred to nitrocellulose and hybridized with freshly labeled probe #4 (tertiaries). Eight plaques giving positive signals were isolated and DNA prepared (100ml liquid cultures for each). The DNA corresponding to each of these 8 clones was digested with EcoRI (to release inserted human genomic DNA from the lambda vector DNA) and the resulting fragments separated by size using electrophoresis on a 0.8% agarose gel, denatured and transferred to nitrocellulose. This was done in quadruplicate and each filter hybridized with ³²P-labeled probe #'s 1, 2, 3 or 4. The filters were used to expose to X-ray film and a single band of about 4.4kb in size was found to hybridize with all tour probes for two clones. These two clones were identical except that one had more insert DNA than the other (clone designations are 23 D for the larger insert of 15.21kb and 11 for the smaller insert of approximately 13kb). The 4.4kb gel isolated EcoRI fragment was subcloned in vectors M13 and pUC-9 (a derivative of pBR322). DNA sequencing by the dideoxy technique on M13 DNA using the synthetic probe #3 and its reverse complement as primers was carried out.

The 4.4kb fragment was partially sequenced and has the following sequence encompassing the probe #4 sequence, indicated in parenthesis, and the partial amino acid sequence of the 67/70kd fragment originally determined, indicated in brackets.

1                                                    10
val ser phe phe arg ala gln arg glu arg leu ser gly asn
GG GTG TCC TTC TTC AGG GCT CAG AGG GAG CGA TTA AGT GGC AAC

                    20
glu ala asn arg pro gly lys leu pro phe leu arg val ala thr
GAA GCA AAC AGG CCT GGA AAA CTT CCC TTT CTG AGA GTA GCA ACA

30                                      40
glu thr leu gln arg leu pro pro ser tyr trp ile leu leu leu
GAA ACT CTG CAA AGA CTC CCT CCA AGC TAT TGG ATC CTC TTG CTT

                    50
gly ile pro leu trp tyr ser glu tyr gln lys lys ser gly lys
GGG ATA CCA CTA TGG TAC TCA GAG TAC CAA AAG AAG AGT GGA AAG

60                                      70
ser gln glu lys ser pro glu lys thr ala phe lys lys lys asp
TCC CAA GAG AAG TCA CCA GAA AAA ACA GCA TTT AAG AAA AAG GAT

                    80
thr ile leu ser leu asn ala cys glu ser asn his ala ile ala
ACC ATT TTG TCC CTG AAC GCT TGT GAA AGC AAT CAT GCA ATA GCA

90                                      100
ala ile asn glu gly gln asn lys pro glu ile glu val thr trp
GCA ATA AAT GAG GGA CAA AAT AAG CCC GAA ATA GAA GTC ACC TGG

                    110
ala lys gln asn arg thr glu arg leu cys ser gln asn pro pro
GCA AAG CAA AAT AGG ACT GAA AGG CTG TGC TCT CAA AAC CCA CCA

120                                     130
val leu lys arg his gln arg glu ile thr arg thr thr leu gln
GTC TTG AAA CGC CAT CAA CGG GAA ATA ACT CGT ACT ACT CTT CAG

                    140
ser asp gln glu glu ile asp tyr asp asp thr ile ser val glu
TCA GAT CAA GAG GAA ATT GAC TAT GAT GAT ACC ATA TCA GTT GAA

150                                     160
met lys lys glu asp phe asp ile tyr asp glu asp glu asn gln
ATG AAG AAG GAA GAT TTT GAC ATT TAT GAT GAG GAT GAA AAT CAG

                    170
ser pro arg ser [phe gln lys lys thr (arg his tyr phe ile ala
AGC CCC CGC AGC [TTT CAA AAG AAA ACA (CGA CAC TAT TTT ATT GCT

180                                     190
ala val glu arg leu trp asp tyr gly met)] ser ser ser pro his
GCA GTG GAG AGG CTC TGG GAT TAT GGG ATG)] AGT AGC TCC CCA CAT

                    200
val leu arg asn arg tyr glu cys ile gly tyr ser phe ala leu
GTT CTA AGA AAC AGG TAT GAA TGC ATT GGT TAT TCC TTT GCT CTG

210 211
leu leu OP
CTC TTG TGA CATTTGACTTTACCAGATGATGACACCAACC

This clone thus corresponds to the gene for the 77/80kd doublet protein, which, as it has been shown above, corresponds in part to the human Factor VIIIC complex.

Clone 23D was subcloned in phage M13 as EcoRI fragments, and the sequence corresponding to the inserted human DNA was determined. The complete 15.121kb sequence of clone 23D sequence is set forth in Appendix A, attached hereto. The subclone designations are given at the right hand margin of the sequence, and refer to the EcoRI-EcoRI fragment extending in the 3'-direction. An open reading frame of 3.110kb was found to exist from the 3'-end of the 70-3 fragment to the middle of the 4.4kb fragment. The open reading frame thus comprises at least part of the coding region for the 77/80kb doublet protein.

### I. Comparison of Genomic DNA with cDNA

Three cDNA clones were obtained as follows. Clone C1 was obtained by screening a human liver cDNA library with a probe constructed from the 4.4kb EcoRI fragment of clone 23 D. Clone C2 was also obtained by screening a human liver cDNA library with the 4.4kb probe. Clone 2-11 was obtained by screening a human kidney cDNA library with a synthetic 45-mer probe based on the DNA sequence found at the 3'-end of the open reading frame of clone 23 D (nucleotides 9391 to 9435 in Appendix A). The probe comprised the non-coding strand of the following sequence:

Ser Pro Arg Ser Phe Gln Lys Lys
5'-AGC CCC CGC AGC TTT CAA AAG AAA
Non-coding- 3'-TCG GGG GCG TCG AAA GTT TTC TTT
(probe)
Thr Arg His Tyr Phe Ile Ala
ACA CGA CAC TAT TTT ATT GCT-3'
TGT GCT GTG ATA AAA TAA CGA-5'

The clones were sequenced and their locations relative to the genomic DNA of clone 23 D determined by comparing the sequences. Clone C1, which is 304kp in length, overlaps with the open reading frame from nucleotide 7773 to 8077, as numbered in Appendix A. Clone C2, which is 878bp in length, partially overlaps with the 3'-end of the open reading frame beginning at nucleotide 9538 and extending beyond nucleotide 9497, which is at the 3'-end of the open reading frame. Clone 2-11, which is 572 bp in length, also overlaps the 3'-end of the open reading frame beginning at nucleotide 9190 and extends beyond its termination. These findings thus confirm that the open reading frame is transcribed.

The coding information derived from the 4.4kb open reading frame may be combined with the additional coding information derived from clones 2-11 and C2 to prepare and extend sequence of 3.841 kb corresponding to the total known coding sequence, as set forth in Appendix B. The regions corresponding to the C1, C2 and 2-11 probes are boxed.

To prepare Factor VIIIC, the DNA sequences from Clone 23 or Clone 11 are inserted into an SV-40 promoter as described by Laub et al., J. Virology (1983) 48:271, so as to be under the control of the SV-40 early promoter. The resulting recombinant plasmid maybe transfected into COS cells (Guzman, supra.). Alternatively, the coding sequence can be inserted into a plasmid, e.g., pBR322, into which has been inserted the long terminal repeats of Maloney murine sarcoma virus, so that the Clone 23 or 11 sequences are under the transcriptional control of the viral regulatory system. The constructs may then be introduced into 3T3 mouse fibroblasts for efficient expression (see Perkins et al., Molecular and Cellular Biology, June 1983, Vol. 3, No. 6, p. 1123).

The above results demonstrate that genomic DNA has been isolated which codes for subunits of the Factor VIIIC complex. By use of this genomic DNA, the DNA may be further manipulated to provide for a sequence encoding for Factor VIIIC complex subunits. The DNA may then be used in an expression vector for production of the Factor VIIIC subunits, which may be used in a variety of ways, for example, as reagents in diagnostic assays, as therapeutic agents, for the production of monoclonal or polyclonal antibodies, which may then be used for the purification of Factor VIIIC complex or other purposes. The genomic DNA sequences may also be used for isolation of mRNA encoding proFactor VIIIC to provide for the precursor protein. This protein may then be administered for in vivo processing.

The subject techniques have provided a DNA sequence which includes the specific sequence at or adjacent to the 5'-terminus of the sequence coding for the 67/70kd doublet, which specific sequence is also present in the 77/80kd doublet, about 200 to 400, more exactly about 275 to 325 bases downstream from the 5'-terminus.

Although the foregoing invention has been described in some detail by way of illustration and example for purposes of clarity of understanding, it will be obvious that certain changes end modifications may be practiced within the scope of the appended claims.

The bacteriophage λFVIII23D containing the 14.43kb EcoRI fragment was deposited at the A.T.C.C. on January 4, 1984 and given Accession No. 40094.

APPENDIX A

1 GAATTCCCCGGATCTCTTATATTCCATTGACTTATTGGTCTATCTTTGTACCAGTAACAT EcoRI #81
CTTAAGGGGCCTAGAGAATATAAGGTAACTGAATAACCAGATAGAAACATGGTCATTGTA

1 ecor1, 10 binI,

61 ACTATCTTATAGTAAGTTTTAATATTTGTTAGTATAATTCCTCCAACTTTGCTCTCTTTG
TGATAGAATATCATTCAAAATTATAAACAATCATATTAAGGAGGTTGAAACGAGAGAAAC

121 ATTTTTATATAAATTTTAGAATCATCTTGTCAATTTTATCAATAAAAATATCTTCTGAGG
TAAAAATATATTTAAAATCTTAGTAGAACAGTTAAAATAGTTATTTTTATAGAAGACTCC

171 mbo11,

181 TTTTGATAGGGATTACATTGATTCTGCAGATCAATTTGGGGGAGCATACCTCTTGACAAT
AAAACTATCCCTAATGTAACTAAGACGTCTAGTTAAACCCCCTCGTATGGAGAACTGTTA

204 pst1,

241 ATTGAGTATTCCTGTCCATGAGCATGGTTTAGTTATTTATTTACATCTTTTAAATTTTCT
TAACTCATAAGGACAGGTACTCGTACCAAATCAATAAATAAATGTAGAAAATTTAAAAGA

256 bstXI, 289 aha111,

301 CTTGGCAATGTTTTTGTAAAATTTTAGTGTACAGGTCTTGCACATTTTTGTAAAGTGTAT
GAACCGTTACAAAAACATTTTAAAATCACATGTCCAGAACGTGTAAAAACATTTCACATA

361 TCTTTGGTATTTAATGCTATTATAATATTTTTTATGGTATCAGTTTTTGTCAATTGTGTT
AGAAACCATAAATTACGATAATATTATAAAAAATACCATAGTCAAAAACAGTTAACACAA

421 TTTCTAGAAATATGTCCCTTTCATCTAAGTTGTTTAATTTACTTGAAGTAAGTTATTTGT
AAAGATCTTTATACAGGGAAAGTAGATTCAACAAATTAAATGAACTTCATTCAATAAACA

423 xba1,

481 AATGTTCTTTTATCATCCTTATAATGTCTGTACTGATGTCTCCGTTTTCATACCTGATAT
TTACAAGAAAATAGTAGGAATATTACAGACATGACTACAGAGGCAAAAGTATGGACTATA

541 TGTTATTTATATTTTATCTATTTTAAGTTTATCGCCTGCACCCACTAACTCGTCATCTAG
ACAATAAATATAAAATAGATAAAATTCAAATAGCGGACGTGGGTGATTGAGCAGTAGATC

601 CATTAGGTATATCTCCCAATGATATCCCTCGCCCCTCCCCCTAGTGCACATGTACCCTAA
GTAATCCATATAGAGGGTTACTATAGGGAGCGGGGAGGGGGATCACGTGTACATGGGATT

621 ecor5,

661 AACTTAAAGTATAATAAAAAAAAAAAGTTTGTCGCCTTTGTTATAGGTTCATCAAATTTA
TTGAATTTCATATTATTTTTTTTTTTCAAACAGCGGAAACAATATCCAAGTAGTTTAAAT

721 CTAATCCTACCATAGGATCAAATTTTTGCTTTGTTCATCAGGTCATGGAATTAAAAAAAT
GATTAGGATGGTATCCTAGTTTAAAAACGAAACAAGTAGTCCAGTACCTTAATTTTTTTA

735 binl,

781 TTTTTTGTTTTGTTTATTTTCTATGTTGAATTTTTATTTTTATTTTGCTTTGCATTTCAT
AAAAAACAAAACAAATAAAAGATACAACTTAAAAATAAAAATAAAACGAAACGTAAAGTA

841 TTATTTCTGGTCTTTTGTTATTATTCCAATACTTTCTCTGGATTCAGTTTTCTTTTTCTT
AATAAAGACCAGAAAACAATAATAAGGTTATGAAAGAGACCTAAGTCAAAAGAAAAAGAA

897 mbo11,

901 CTTGACTTCTTGAGATTAAAACTTGGTTCATTGATTTTCAACATTTCTTACTTTTTAAAA
GAACTGAAGAACTCTAATTTTGAACCAAGTAACTAAAAGTTGTAAAGAATGAAAAATTTT

954 aha111,

961 GTTCATACAAAGCATAAATTTCCCTTTATATACTGCTTTAACCCCATATCACAAATTTTG
CAAGTATGTTTCGTATTTAAAGGGAAATATATGACGAAATTGGGGTATAGTGTTTAAAAC

1021 ATGAGTCATATTTTTATTATCATTGAGTTTAAAATATTTTATAAATTTTACGTTATTTCT
TACTCAGTATAAAAATAATAGTAACTCAAATTTTATAAAATATTTAAAATGCAATAAAGA

1048 aha111,

1081 TGTTTTATTCATAGTTATTTTACAGTGTTTTACTTAATTTCCCAATATATGGCGATTTTC
ACAAAATAAGTATCAATAAAATGTCACAAAATGAATTAAAGGGTTATATACCGCTAAAAG

1141 TGGCTCTCTCTCTGTTGCTGATTTATTTGTTTTACTGTGATCACATAACATACTCTATAT
ACCGAGAGAGAGACAACGACTAAATAAACAAAATGACACTAGTGTATTGTATGAGATATA

1178 bcl1,

1201 TATTTCAATCATTTGAAATTTGTTGAGACTTGCTATATGCCCAGTGAATAATTTAATTTG
ATAAAGTTAGTAAACTTTAAACAACTCTGAACGATATACGGGTCACTTATTAAATTAAAC

1261 ATTAATGGCCCACTGCATTTGAAACAAATATGTCTTTGCTGTTATTGGGTAGAGTGTCAT
TAATTACCGGGTGACGTAAACTTTGTTTATACAGAAACGACAATAACCCATCTCACAGTA

1321 ATATTTGCCTATTATGTCAAGTTGCTTTATCATGTTTTTAAAATCCTTATAAACTTCCTG
TATAAACGGATAATACAGTTCAACGAAATAGTACAAAAATTTTAGGAATATTTGAAGGAC

1357 aha111,

1381 ATATTTTATCTGTTTCTTACAGACATGGAGGAAACTGTGACATCTCTGTGATTGTTGAGT
TATAAAATAGACAAAGAATGTCTGTACCTCCTTTGACACTGTAGAGACACTAACAACTCA

1441 TTGTTATTTTGATTTTTGTATTTGTTAGTAAGAATGTTTCAAAATAAATGAGGCAATATA
AACAATAAAACTAAAAACATAAACAATCATTCTTACAAAGTTTTATTTACTCCGTTATAT

1501 AAATCAGGTGCTTACAACTTTAAGGTTGTATTCTTATTAAATCAATTCTTTCATCTTTGT
TTTAGTCCACGAATGTTGAAATTCCAACATAAGAATAATTTAGTTAAGAAAGTAGAAACA

1561 GAAATGTCCTTTTTGTCTGAAGTAGTACTTGTTGCCTTAAACGTCTGCATTGTCTAAAAT
CTTTACAGGAAAAACAGACTTCATCATGAACAACGGAATTTGCAGACGTAACAGATTTTA

1584 sca1,

1621 TTATACACCTGCACTATCATATTTATTTTGCTTAATGTTTGTGTGGTATATATTTCCCAT
AATATGTGGACGTGATAGTATAAATAAAACGAATTACAAACACACCATATATAAAGGGTA

1681 TCTTTTCTTTTCAACCTATCTCTATCCTTATATTTACAGTGTGTCTTTTTTAACATTTTC
AGAAAAGAAAAGTTGGATAGAGATAGGAATATAAATGTCACACAGAAAAAATTGTAAAAG

1741 TGTAGTGCAAGTCTGTTGGTTAATTAATTTTGTCAGTTTTTGCTTCTCTGAAAAGTCTTT
ACATCACGTTCAGACAACCAATTAATTAAAACAGTCAAAAACGAAGAGACTTTTCAGAAA

1801 ATTTCTGTTTTTGAATTATATCTTCACTGTGTATAGAATTCTGGGTTTGTGGTTGTTGTT EcoRI 61
TAAAGACAAAAACTTAATATAGAAGTGACACATATCTTAAGACCCAAACACCAACAACAA

1821 mbo11, 1836 ecor1,

1861 TTTGTTTTATTACATTTTGTTTTATTGGTTTTTCCCTCAGCACTTTAAAAATGCCGCTCC
AAACAAAATAATGTAAAACAAAATAACCAAAAAGGGAGTCGTGAAATTTTTACGGCGAGG

1904 aha111,

1921 ATTGTTTCTACTTGCATAGTTTTTCACAATAAGTTTGTTGTAATTCCCATTTGCATTCAA
TAACAAAGATGAACGTATCAAAAAGTGTTATTCAAACAACATTAAGGGTAAACGTAAGTT

1981 CTGTACATAATGTATCTTTTTAAAAAAATCTGGATGGCTTCAAGCTCTTTACTTTGATTT
GACATGTATTACATAGAAAAATTTTTTTAGACCTACCGAAGTTCGAGAAATGAAACTAAA

1999 aha111,

2041 TTGTGTGTGTGTGTGTGTGTGTGTGTGTGTGTGTGTGTGTGTGTTACTTTGGCATTCTCTAG
AACACACACACACACACACACACACACACACACACACACACACAATGAAACCGTAAGAGATC

2101 GCTTATTCAATCTGTAATTTGGTATCATTAATTTTGAAAAATATTTATCATCTGTGTCTT
CGAATAAGTTAGACATTAAACCATAGTAATTAAAACTTTTTATAAATAGTAGACACAGAA

2157 mbo11,

2161 CAAATATTGCTTCTAATTCTTTCTCTTCTTCTGGTATTGCATTTACATATATTATACTGT
GTTTATAACGAAGATTAAGAAAGAGAAGAAGACCATAACGTAAATGTATATAATATGACA

2184 mbo11, 2187 mbo11,

2221 TCAATTTTGTCACACAGCTCTTGAATATGCTGTTCTAGTTTGTTTTCTAACTATTTTTCC
AGTTAAAACAGTGTGTCGAGAACTTATACGACAAGATCAAACAAAAGATTGATAAAAAGG

2281 TTCTAGTTGTTCAGTTTTGTTGACATTTATTGACCTATTTTTACTTCAGTAATTGTTTCC
AAGATCAACAAGTCAAAACAACTGTAAATAACTGGATAAAAATGAAGTCATTAACAAAGG

2341 TGGGTTGTTGAGTTAATTGATGAACTCATAGATGACATACAATTTATCTTTTTATTCTTG
ACCCAACAACTCAATTAACTACTTGAGTATCTACTGTATGTTAAATAGAAAAATAAGAAC

2401 TGTCACTTGTGCTTTTGATGTCACATCTAGGAAGGCTTTGTCTAGCCCAAGGCCACATTT
ACAGTGAACACGAAAACTACAGTGTAGATCCTTCCGAAACAGATCGGGTTCCGGTGTAAA

2461 ACTTCCATATTTTCTTCTAAGAGTTTGATAGTTGTAGCTGTTACATATATGTCTATGATC
TGAAGGTATAAAAGAAGATTCTCAAACTATCAACATCGACAATGTATATACAGATACTAG

2473 mboii,

2521 TAGTTTGAATAAATTTTTGTGTATGGTGTGAAGAAGAGGTCTAACCTCTTTTTATTTTT
ATCAAACTTATTTAAAAACACATACCACACTTCTTCTCCAGATTGGAGAAAAATAAAAA

2550 mboii, 2553 mboii,

2581 TGCATGTAAACATCCAGTTGTCCCGGCACCATTTGTTGAAAATATGATTCTTTTCCCATT
ACGTACATTTGTAGGTCAACAGGGCCGTGGTAAACAACTTTTATACTAAGAAAAGGGTAA

2641 AAGTCATCTTGACACCCTTGTTGAAAATCAATTGACCATAAATGTGAGAGTTTATTTCTG
TTCAGTAGAACTGTGGGAACAACTTTTAGTTAACTGGTATTTACACTCTCAAATAAAGAC

2701 AACTCTCCATTCTGTTCCATTGACCCATACGTCTTCTATAAATTCCTTATAGCTAATTCT
TTGAGAGGTAAGACAAGGTAACTGGGTATGCAGAAGATATTTAAGGAATATCGATTAAGA

2732 mboii,

2761 ACATTAGACTTTGGTTTTTCAGATGTGGTCATCTCATTAGATTATGGTGATATGTTTGGT
TGTAATCTGAAACCAAAAAGTCTACACCAGTAGAGTAATCTAATACCACTATACAAACCA

2821 TACAAGTTTTATCTTCTCCTTGGCAACATTTTTTTATTACTGCTCTTCTTTTATTCGTT
ATGTTCAAAATAGAAGAGGAACCGTTGTAAAAAAATAATGACGAGAAGAAAATAAGCAA

2832 mboii, 2865 mboii,

2881 TTTGTTGTTCCTTTCTCTTTTTGTGAGACAGGGTCTCACTCTGTTGCCCAGGGTGGAGTA
AAACAACAAGGAAAGAGAAAAACACTCTGTCCCAGAGTGAGACAACGGGTCCCACCTCAT

2907 tthIIIi,

2941 AGTGGTGTGATCATGGCTCACTGCAGCCTCAACCTCCTGGGCTCAAGCAATCCTCCCACC
TCACCACACTAGTACCGAGTGACGTCGGAGTTGGAGGACCCGAGTTCGTTAGGAGGGTGG

2948 bcli, 2961 psti,

3001 TCAGCCTCCCAAGTAGTGGGACTACAGGCATGCACCATCATGCCAGGCTAACTTTTTTAT
AGTCGGAGGGTTCATCACCCTGATGTCCGTACGTGGTAGTACGGTCCGATTGAAAAAATA

3028 sphi,

3061 TTTTTGTAGAGACAGGGTCTTGCTTTGTTGCCCAGGCTGGTCTTGAACTCCAAGCTCAAG
AAAAACATCTCTGTCCCAGAACGAAACAACGGGTCCGACCAGAACTTGAGGTTCGAGTTC

3071 tthIIIi,

3121 CAATCCTCCAACCTCAGCTTCCCAAAGTGCTGGGATTACCAGTATGAGCCACTGCACCTG
GTTAGGAGGTTGGAGTCGAAGGGTTTCACGACCCTAATGGTCATACTCGGTGACGTGGAC

3142 bstXI,

3181 GCCTTGTTGTTCCTTTCTTCCTTTTATCTTGTAGTATCTTGATCCTTTTTGCTTATTATT
CGGAACAACAAGGAAAGAAGGAAAATAGAACATCATAGAACTAGGAAAAACGAATAATAA

3196 mboii,

3241 TATCATTGAGGTGAGTTTGTTCTAAATAAACTTTTCGCAGAAAGTTTATGTAGATATATT
ATAGTAACTCCACTCAAACAAGATTTATTTGAAAAGCGTCTTTCAAATACATCTATATAA

3301 AATATCAATGAGGCATTAGGAGTGAGTTTTAGATTAGCAGGAATTCTCTTATGAAAGGTA EcoRI #79
TTATAGTTACTCCGTAATCCTCACTCAAAATCTAATCGTCCTTAAGAGAATACTTTCCAT

3341 ecori,

3361 TTGATTGTATGTGTGAGTTTTCAGCCTTTACTTCTCTCCAGCCCATCAAGATCAAAGCTG
AACTAACATACACACTCAAAAGTCGGAAATGAAGAGAGGTCGGGTAGTTCTAGTTTCGAC

3418 psti,

3421 CAGAGTTGTTTACATTTCAGAATTTCTTTCCTCCCCTCCCCACCTTGCAAAACAGACTTA
GTCTCAACAAATGTAAAGTCTTAAAGAAAGGAGGGGAGGGGTGGAACGTTTTGTCTGAAT

3481 CAGTGCAAAAATGACTTGTCAATGTGATTCTTTCTTCTGGTCCCATCTTTCCTACCTTTT
GTCACGTTTTTACTGAACAGTTACACTAAGAAAGAAGACCAGGGTAGAAAGGATGGAAAA

3513 mbo11,

3541 GAGACTTGTGTTGAAAGGAGTCCAGTAAAACCACCGATGCTAGCTTGTATACCCTGATCC
CTCTGAACACAACTTTCCTCAGGTCATTTTGGTGGCTACGATCGAACATATGGGACTAGG

3601 CATTGTTATAATGAATGACTGACTGTTTTTCATATCAAAGTATAATACTTGCACCTTGAT
GTAACAATATTACTTACTGACTGACAAAAAGTATAGTTTCATATTATGAACGTGGAACTA

3661 AGAAGAAAGAAACAAGGCCAGGCACAGTGGTTCATGCCTGTAATCCCAGCACTTTGGGAG
TCTTCTTTCTTTGTTCCGGTCCGTGTCACCAAGTACGGACATTAGGGTCGTGAAACCCTC

3662 mbo11, 3706 bstXI,

3721 GCTGAAGTAGGAGGAATAATTAGACTTTGATATTAAAAATGGGGAAGATATGGCGGTATA
CGACTTCATCCTCCTTATTAATCTGAAACTATAATTTTTACCCCTTCTATACCGCCATAT

3764 mbo11,

3781 AGATATGAGGAAGTATGTCTTCTTGGACTTTTCTGAAATCTGCCAGCCATGAGCATCCAC
TCTATACTCCTTCATACAGAAGAACCTGAAAAGACTTTAGACGGTCGGTACTCGTAGGTG

3798 mbo11,

3841 TCTTTTTATCTTTTGATACCTGTTTTATATTAACTATACTTCTATTTTGTGGTTTCATGG
AGAAAAATAGAAAACTATGGACAAAATATAATTGATATGAAGATAAAACACCAAAGTACC

3901 CTTCAGATATCTTCCAGCTTAATTGTACTCTGTCTTTAATTATTTGCAGCAATTTCTGAA
GAAGTCTATAGAAGGTCGAATTAACATGAGACAGAAATTAATAAACGTCGTTAAAGACTT

3906 ecor5, 3910 mbo11,

3961 AGGAGCAAGAAGAAAGGCCAGCCCTTTCTTCACTACCTTAAAAACTGGTCATCTTTTGAA
TCCTCGTTCTTCTTTCCGGTCGGGAAAGAAGTGATGGAATTTTTGACCAGTAGAAAACTT

3969 mbo11, 3987 mbo11,

4021 ACCCCATGAAAGGCACAGTGGGTGACCATCTACATCAGGAGTGGGCAAACTATGGCCCTT
TGGGGTACTTTCCGTGTCACCCACTGGTAGATGTAGTCCTCACCCGTTTGATACCGGGAA

4041 bstE2,

4081 GGGTAGTCTGGTCTCTGTCCGTTTTTGTAAGGTTTTATTGGAACCCATACACACACAAAC
CCCATCAGACCAGAGACAGGCAAAAACATTCCAAAATAACCTTGGGTATGTGTGTGTTTG

4141 TGATCATCTCTTGTTATGTGTTAAGTTTTCTTATAATTATTCTTTTCTATTTCATCCAGC
ACTAGTAGAGAACAATACACAATTCAAAAGAATATTAATAAGAAAAGATAAAGTAGGTCG

4141 bcl1, 4197 pvu11,

4201 TGAATTACCATTTCACCTTGTGTATGCCCTTTCCAGTCTTAAATTATAGAAGCTATGTCT
ACTTAATGGTAAAGTGGAACACATACGGGAAAGGTCAGAATTTAATATCTTCGATACAGA

4261 TTGTGGTTTTAAATTATGGAAGCTGTGCCTTCTTGTGTTTTAGTATGGCAAGATATTTGT
AACACCAAAATTTAATACCTTCGACACGGAAGAACACAAAATCATACCGTTCTATAAACA

4268 aha111,

4321 TCTTACTTTTCCTCTAGAATCTGCAGTGGATTACTTTCAGAAGTAGGTATTGCCTCTAAG
AGAATGAAAAGGAGATCTTAGACGTCACCTAATGAAAGTCTTCATCCATAACGGAGATTC

4333 xba1, 4341 pst1,

4381 TTTCCTGACTTCTGTTTCCTTCTCCCTGTTCTGTAGTATATTTATAGATTGCATGTTTTC
AAAGGACTGAAGACAAAGGAAGAGGGACAAGACATCATATAAATATCTAACGTACAAAAG

4441 TCGTCCTTAAACAAGGTACAAATATGTTCTGATCAAGGTTTGCTAGCCGAGAGGATAAGT
AGCAGGAATTTGTTCCATGTTTATACAAGACTAGTTCCAAACGATCGGCTCTCCTATTCA

4470 bcl1,

4501 GGCTTGCTCTTAGCCTAACTCTCTGTCTACTTAATGGATGTCGAATTCCCTTCTCAACTC EcoRI #70-3
CCGAACGAGAATCGGATTGAGAGACAGATGAATTACCTACAGCTTAAGGGAAGAGTTGAG

4543 ecor1,

4561 AACAGCTAGTAACTAGTTCCAAGATCGGATAATAGCTTACAGAATTAATTAGTTCAGTGA
TTGTCGATCATTGATCAAGGTTCTAGCCTATTATCGAATGTCTTAATTAATCAAGTCACT

4621 AAGCCCTGGTTTGATCACAGAGGCAGGAGACTGATCTATATATTTGTAGTGCTGACTAGA
TTCGGGACCAAACTAGTGTCTCCGTCCTCTGACTAGATATATAAACATCACGACTGATCT

4632 bcl1,

4681 CCCCAACTTGGCCAGATAGCTTATACCTATGTCAGAAGTTGGAGGAGGAGATTGACAGCT
GGGGTTGAACCGGTCTATCGAATATGGATACAGTCTTCAACCTCCTCCTCTAACTGTCGA

4689 bal1, 4736 pvu11,

4741 GAGGTGTCTCCAAGGGAATTACAATTATTTCATTTTCTTTGTGTCTGTAATTATGTCTC
CTCCACAGAGGTTCCCTTAATGTTAATAAAGTAAAAGAAACACAGACATTAATACAGAG

4801 TTTTCTTACTTCAAATTTTGTATATGTGAAATTTCTTAATTTTTATTTGGTTATTTATTT
AAAAGAATGAAGTTTAAAACATATACACTTTAAAGAATTAAAAATAAACCAATAAATAAA

4861 TATTGACTTTTTCATCAAACAACCAACTCTAAGTTCTTGGATTGAAATTTATGAATTAAT
ATAACTGAAAAAGTAGTTTGTTGGTTGAGATTCAAGAACCTAACTTTAAATACTTAATTA

4921 TATAATGTTTTAAATTTACCATTAGTCACACATACAATACAAAAAGATATTTTCATTACA
ATATTACAAAATTTAAATGGTAATCAGTGTGTATGTTATGTTTTTCTATAAAAGTAATGT

4929 aha111,

4981 AAATTTTAACCCAAGCCATTAAAACTAAACTCCAGCTTGGTCACCATTCCTAATACCAGG
TTTAAAATTGGGTTCGGTAATTTTGATTTGAGGTCGAACCAGTGGTAAGGATTATGGTCC

5019 bstE2,

5041 CCCCTCTCAGAGGAGGGTAAATAAAAGTCCATTTTGGTAACTAGTTTGACGAGAATCTCT
GGGGAGAGTCTCCTCCCATTTATTTTCAGGTAAAACCATTGATCAAACTGCTCTTAGAGA

5101 AATTGTTTCCCTGTTTTTCTTATAATGTCTGCCTTTGAATTATCCTCTTTTTTTCAAGAG
TTAACAAAGGGACAAAAAGAATATTACAGACGGAAACTTAATAGGAGAAAAAAAGTTCTC

5161 TTCCATCATATAATGTATTCCTTTTTGCTTACAGATTGACCTCCTAGAGGCATGCATCCC
AAGGTAGTATATTACATAAGGAAAAACGAATGTCTAACTGGAGGATCTCCGTACGTAGGG

5210 sph1, 5212 ava3,

5221 CCTGCTCCAGTCTGAGCTGGTTCTCTCTAGGCCTGAGGCTCAGTTATCAACTTGGGACTT
GGACGAGGTCAGACTCGACCAAGAGAGATCCGGACTCCGAGTCAATAGTTGAACCCTGAA

5222 tth1111, 5249 stu1, 5252 mstII,

5281 TTATTTACCCTCCCTCAGGATAGGTACCTGGTTCTTGGATACCAGGTCTTACTCTTGTTT
AATAAATGGGAGGGAGTCCTATCCATGGACCAAGAACCTATGGTCCAGAATGAGAACAAA

5293 mstII, 5303 kpn1,

5341 GAGATACTCCTTTATTTTGGTGGCAAACATTCCATAATCACTTCCTGATAATGGTGACAT
CTCTATGAGGAAATAAAACCACCGTTTGTAAGGTATTAGTGAAGGACTATTACCACTGTA

5401 TTGAGGTAGATTTTCTGAGTCTTTATATGTTAGAATATGTTTTTATTCTCCTTTGCCACT
AACTCCATCTAAAAGACTCAGAAATATACAATCTTATACAAAAATAAGAGGAAACGGTGA

61 TGATTGATAATTTGGCTGCACATAGAATGTAAGCATTCACAGGGCTTTTGGGAGCAGCTT
ACTAACTATTAAACCGACGTGTATCTTACATTCGTAAGTGTCCCGAAAACCCTCGTCGAA

5521 CCCTTGAGAAGAAGGTGGATGCAGTTAAGATCTTCCCTGAAAAGACTGTGTGAAATGACA
GGGAACTCTTCTTCCACCTACGTCAATTCTAGAAGGGACTTTTCTGACACACTTTACTGT

5528 mbo11, 5548 bgl11, 5551 mbo11,

5581 AGGCTATTGAGGTACCCAACGGGTAGCTTGGTAAAGGTGTATCTTGTCTCTTATGTTTAT
TCCGATAACTCCATGGGTTGCCCATCGAACCATTTCCACATAGAACAGAGAATACAAATA

5591 kpn1, 5639 ava3,

5641 GCATCTACTTTACCACCATGTAATTAGCCTATTATAACTTGTTGCATTCCTTCACTCAGC
CGTAGATGAAATGGTGGTACATTAATCGGATAATATTGAACAACGTAAGGAAGTGAGTCG

5701 TAATCTTGTTCATGGTAGAAATTAATATTGCTAGTACCATGGTAGAATTTTATATTAGCT
ATTAGAACAAGTACCATCTTTAATTATAACGATCATGGTACCATCTTAAAATATAATCGA

573[?] n[?][?]1,

5761 GGATCCTAGACCTAAATGCTCCTTCCTTGTGCTTACATATTCTGCAAGTGGGTGACAGTG
CCTAGGATCTGGATTTACGAGGAAGGAACACGAATGTATAAGACGTTCACCCACTGTCAC

5761 bamh1 binI,

5821 GATGCTAGGTATTGTGTTATGTGATTTCAATGTATTATATGATGGGGTAGATACTATTAG
CTACGATCCATAACACAATACACTAAAGTTACATAATATACTACCCCATCTATGATAATC

5881 CCTCATTTGAAAATTAGAAACCTATTGTTCAGAAAGTTTAAGTGACTTTCTCAAAGTCAC
GGAGTAAACTTTTAATCTTTGGATAACAAGTCTTTCAAATTCACTGAAAGAGTTTCAGTG

5941 ACAGCTGGTAAATGGTAGAACTGGGATTTGAATCCAGGCAATCTGATGGATTTAAGAGGA
TGTCGACCATTTACCATCTTGACCCTAAACTTAGGTCCGTTAGACTACCTAAATTCTCCT

5942 pvu11,

6001 ACCTGTGCCACTATGTGATATAGCAATAACATCTAGCCAATATAAATACTTTCCTGAATT
TGGACACGGTGATACACTATATCGTTATTGTAGATCGGTTATATTTATGAAAGGACTTAA

6061 AAATCACTGTCATATTCAGAGAGTTCTGTGTCACTATTAAGACCCTCCATTATTGTGTAT
TTTAGTGACAGTATAAGTCTCTCAAGACACAGTGATAATTCTGGGAGGTAATAACACATA

6063 ecopDXI,

6121 ATTAATTACATTTCCCTATTTTAATCCCAATATCTATTCATTTTTATCACAGTCCTTTCT
TAATTAATGTAAAGGGATAAAATTAGGGTTATAGATAAGTAAAAATAGTGTCAGGAAAGA

6181 TATGGTCACAAACAGGCATAGTACAACAGCAGCAATGCAAAAACCACTTTTATATTACAT
ATACCAGTGTTTGTCCGTATCATGTTGTCGTCGTTACGTTTTTGGTGAAAATATAATGTA

6241 GTTCCAGGGATTTAACCCAATGACCTGTGATATAATGATACTGACTAGTATTTTTTCATT
CAAGGTCCCTAAATTGGGTTACTGGACACTATATTACTATGACTGATCATAAAAAAGTAA

6301 TATCTGGGAATGGGAGAGAACCTCTAACAGAACGTTTTAGAATCTGTGTTATGAGTAACC
ATAGACCCTTACCCTCTCTTGGAGATTGTCTTGCAAAATCTTAGACACAATACTCATTGG

6361 AGAGTCTTAGTTCTTCCTCATCTCCAGGTCTATGGATTCTGGGGTGCCACAACTCAGACT
TCTCAGAATCAAGAAGGAGTAGAGGTCCAGATACCTAAGACCCCACGGTGTTGAGTCTGA

6372 mboi1, 6384 bstXI,

6421 TTCGGAAGCAGAGGCATGACCGCCTTACTGAAGGTTTCTAGTTGTGACAAGAACACTGGT
AAGCCTTCGTCTCCGTACTGGCGGAATGACTTCCAAAGATCAACACTGTTCTTGTGACCA

6481 GATTATTACGAGGACAGTTATGAAGATATTTCAGCATACTTGCTGAGTAAAAACAATGCC
CTAATAATGCTCCTGTCAATACTTCTATAAAGTCGTATGAACGACTCATTTTTGTTACGG

6502 mboi1,

6541 ATTGAACCAAGAAGCTTCTCCCAGAATTCAAGACACCCTAGCACTAGGCAAAAGCAATTT 4.3kbEcoRI
TAACTTGGTTCTTCGAAGAGGGTCTTAAGTTCTGTGGGATCGTGATCCGTTTTCGTTAAA

6552 hind111, 6564 ecor1,

6601 AATGCCACCACAATTCCAGAAAATGACATAGAGAAGACTGACCCTTGGTTTGCACACAGA
TTACGGTGGTGTTAAGGTCTTTTACTGTATCTCTTCTGACTGGGAACCAAACGTGTGTCT

6633 mboi1,

6661 ACACCTATGCCTAAAATACAAAATGTCTCCTCTAGTGATTTGTTGATGCTCTTGCGACAG
TGTGGATACGGATTTTATGTTTTACAGAGGAGATCACTAAACAACTACGAGAACGCTGTC

6716 tthIII1,

6721 AGTCCTACTCCACATGGGCTATCCTTATCTGATCTCCAAGAAGCCAAATATGAGACTTTT
TCAGGATGAGGTGTACCCGATAGGAATAGACTAGAGGTTCTTCGGTTTATACTCTGAAAA

6781 TCTGATGATCCATCACCTGGAGCAATAGACAGTAATAACAGCCTGTCTGAAATGACACAC
AGACTACTAGGTAGTGGACCTCGTTATCTGTCATTATTGTCGGACAGACTTTACTGTGTG

6841 TTCAGGCCACAGCTCCATCACAGTGGGGACATGGTATTTACCCCTGAGTCAGGCCTCCAA
AAGTCCGGTGTCGAGGTAGTGTCACCCCTGTACCATAAATGGGGACTCAGTCCGGAGGTT

6855 bstXI, 6884 tthIII1, 6891 stu1,

6901 TTAAGATTAAATGAGAAACTGGGGACAACTGCAGCAACAGAGTTGAAGAAACTTGATTTC
AATTCTAATTTACTCTTTGACCCCTGTTGACGTCGTTGTCTCAACTTCTTTGAACTAAAG

6929 pst1, 6945 mboi1,

6961 AAAGTTTCTAGTACATCAAATAATCTGATTTCAACAATTCCATCAGACAATTTGGCAGCA
TTTCAAAGATCATGTAGTTTATTAGACTAAAGTTGTTAAGGTAGTCTGTTAAACCGTCGT

7021 GGTACTGATAATACAAGTTCCTTAGGACCCCCAAGTATGCCAGTTCATTATGATAGTCAA
CCATGACTATTATGTTCAAGGAATCCTGGGGGTTCATACGGTCAAGTAATACTATCAGTT

7040 mstII,

7081 TTAGATACCACTCTATTTGGCAAAAAGTCATCTCCCCTTACTGAGTCTGGTGGACCTCTG
AATCTATGGTGAGATAAACCGTTTTTCAGTAGAGGGGAATGACTCAGACCACCTGGAGAC

7141 AGCTTGAGTGAAGAAAATAATGATTCAAAGTTGTTAGAATCAGGTTTAATGAATAGCCAA
TCGAACTCACTTCTTTTATTACTAAGTTTCAACAATCTTAGTCCAAATTACTTATCGGTT

7150 mboII,

7201 GAAAGTTCATGGGGAAAAAATGTATCGTCAACAGAGAGTGGTAGGTTATTTAAAGGGAAA
CTTTCAAGTACCCCTTTTTTACATAGCAGTTGTCTCTCACCATCCAATAAATTTCCCTTT

7249 ahaIII,

7261 AGAGCTCATGGACCTGCTTTGTTGACTAAAGATAATGCCTTATTCAAAGTTAGCATCTCT
TCTCGAGTACCTGGACGAAACAACTGATTTCTATTACGGAATAAGTTTCAATCGTAGAGA

7262 sacI,

321 TTGTTAAAGACAAACAAAACTTCCAATAATTCAGCAACTAATAGAAAGACTCACATTGAT
AACAATTTCTGTTTGTTTTGAAGGTTATTAAGTCGTTGATTATCTTTCTGAGTGTAACTA

7381 GGCCCATCATTATTAATTGAGAATAGTCCATCAGTCTGGCAAAATATATTAGAAAGTGAC
CCGGGTAGTAATAATTAACTCTTATCAGGTAGTCAGACCGTTTTATATAATCTTTCACTG

7408 bstXI,

7441 ACTGAGTTTAAAAAAGTGACACCTTTGATTCATGACAGAATGCTTATGGACAAAAATGCT
TGACTCAAATTTTTTCACTGTGGAAACTAAGTACTGTCTTACGAATACCTGTTTTTACGA

7447 ahaIII,

7501 ACAGCTTTGAGGCTAAATCATATGTCAAATAAAACTACTTCATCAAAAAACATGGAAATG
TGTCGAAACTCCGATTTAGTATACAGTTTATTTTGATGAAGTAGTTTTTTGTACCTTTAC

7519 ndeI,

7561 GTCCAACAGAAAAAAGAGGGCCCCATTCCACCAGATGCACAAAATCCAGATATGTCGTTC
CAGGTTGTCTTTTTTCTCCCGGGGTAAGGTGGTCTACGTGTTTTAGGTCTATACAGCAAG

7578 apaI,

7621 TTTAAGATGCTATTCTTGCCAGAATCAGCAAGGTGGATACAAAGGACTCATGGAAAGAAC
AAATTCTACGATAAGAACGGTCTTAGTCGTTCCACCTATGTTTCCTGAGTACCTTTCTTG

7681 TCTCTGAACTCTGGGCAAGGCCCCAGTCCAAAGCAATTAGTATCCTTAGGACCAGAAAAA
AGAGACTTGAGACCCGTTCCGGGGTCAGGTTTCGTTAATCATAGGAATCCTGGTCTTTTT

7724 mstII,

'741 TCTGTGGAAGGTCAGAATTTCTTGTCTGAGAAAAACAAAGTGGTAGTAGGAAAGGGTGAA
AGACACCTTCCAGTCTTAAAGAACAGACTCTTTTTGTTTCACCATCATCCTTTCCCACTT

7801 TTTACAAAGGACGTAGGACTCAAAGAGATGGTTTTTCCAAGCAGCAGAAACCTATTTCTT
AAATGTTTCCTGCATCCTGAGTTTCTCTACCAAAAAGGTTCGTCGTCTTTGGATAAAGAA

7861 ACTAACTTGGATAATTTACATGAAAATAATACACACAATCAAGAAAAAAAAATTCAGGAA
TGATTGAACCTATTAAATGTACTTTTATTATGTGTGTTAGTTCTTTTTTTTTAAGTCCTT

7918 mboII,

7921 GAAATAGAAAAGAAGGAAACATTAATCCAAGAGAATGTAGTTTTGCCTCAGATACATACA
CTTTATCTTTTCTTCCTTTGTAATTAGGTTCTCTTACATCAAAACGGAGTCTATGTATGT

7981 GTGACTGGCACTAAGAATTTCATGAAGAACCTTTTCTTACTGAGCACTAGGCAAAATGTA
CACTGACCGTGATTCTTAAAGTACTTCTTGGAAAAGAATGACTCGTGATCCGTTTTACAT

8004 mboII, 8007 xmnI,

8041 GAAGGTTCATATGACGGGGCATATGCTCCAGTACTTCAAGATTTTAGGTCATTAAATGAT
CTTCCAAGTATACTGCCCCGTATACGAGGTCATGAAGTTCTAAAATCCAGTAATTTACTA

[?][?][?][?] [?]d[?]I, 8050 ndeI, 8073 scaI,

8101 TCAACAAATAGAACAAAGAAACACACAGCTCATTTCTCAAAAAAAGGGGAGGAAGAAAAC
AGTTGTTTATCTTGTTTCTTTGTGTGTCGAGTAAAGAGTTTTTTTCCCCTCCTTCTTTTG

8152 mbo11,

8161 TTGGAAGGCTTGGGAAATCAAACCAAGCAAATTGTAGAGAAATATGCATGCACCACAAGG
AACCTTCCGAACCCTTTAGTTTGGTTCGTTTAACATCTCTTTATACGTACGTGGTGTTCC

8204 ava3, 8206 sph1, 8220 ecor5,

8221 ATATCTCCTAATACAAGCCAGCAGAATTTTGTCACGCAACGTAGTAAGAGAGCTTTGAAA
TATAGAGGATTATGTTCGGTCGTCTTAAAACAGTGCGTTGCATCATTCTCTCGAAACTTT

8277 xmn1,

8281 CAATTCAGACTCCCACTAGAAGAAACAGAACTTGAAAAAAGGATAATTGTGGATGACACC
GTTAAGTCTGAGGGTGATCTTCTTTGTCTTGAACTTTTTTCCTATTAACACCTACTGTGG

8299 mbo11,

8341 TCAACCCAGTGGTCCAAAAACATGAAACATTTGACCCCGAGCACCCTCACACAGATAGAC
AGTTGGGTCACCAGGTTTTTGTACTTTGTAAACTGGGGCTCGTGGGAGTGTGTCTATCTG

8376 ava1,

8401 TACAATGAGAAGGAGAAAGGGGCCATTACTCAGTCTCCCTTATCAGATTGCCTTACGAGG
ATGTTACTCTTCCTCTTTCCCCGGTAATGAGTCAGAGGGAATAGTCTAACGGAATGCTCC

8461 AGTCATAGCATCCCTCAAGCAAATAGATCTCCATTACCCATTGCAAAGGTATCATCATTT
TCAGTATCGTAGGGAGTTCGTTTATCTAGAGGTAATGGGTAACGTTTCCATAGTAGTAAA

8485 bgl11,

8521 CCATCTATTAGACCTATATATCTGACCAGGGTCCTATTCCAAGACAACTCTTCTCATCTT
GGTAGATAATCTGGATATATAGACTGGTCCCAGGATAAGGTTCTGTTGAGAAGAGTAGAA

8569 mbo11, 8577 mbo11,

8581 CCAGCAGCATCTTATAGAAAGAAAGATTCTGGGGTCCAAGAAAGCAGTCATTTCTTACAA
GGTCGTCGTAGAATATCTTTCTTTCTAAGACCCCAGGTTCTTTCGTCAGTAAAGAATGTT

8641 GGAGCCAAAAAAAATAACCTTTCTTTAGCCATTCTAACCTTGGAGATGACTGGTGATCAA
CCTCGGTTTTTTTTATTGGAAAGAAATCGGTAAGATTGGAACCTCTACTGACCACTAGTT

8694 bcl1,

8701 AGAGAGGTTGGCTCCCTGGGGACAAGTGCCACAAATTCAGTCACATACAAGAAAGTTGAG
TCTCTCCAACCGAGGGACCCCTGTTCACGGTGTTTAAGTCAGTGTATGTTCTTTCAACTC

8761 AACACTGTTCTCCCGAAACCAGACTTGCCCAAAACATCTGGCAAAGTTGAATTGCTTCCA
TTGTGACAAGAGGGCTTTGGTCTGAACGGGTTTTGTAGACCGTTTCAACTTAACGAAGGT

8809 xmn1,

8821 AAAGTTCACATTTATCAGAAGGACCTATTCCCTACGGAAACTAGCAATGGGTCTCCTGGC
TTTCAAGTGTAAATAGTCTTCCTGGATAAGGGATGCCTTTGATCGTTACCCAGAGGACCG

8877 bal1,

8881 CATCTGGATCTCGTGGAAGGGAGCCTTCTTCAGGGAACAGAGGGAGCGATTAAGTGGAAT
GTAGACCTAGAGCACCTTCCCTCGGAAGAAGTCCCTTGTCTCCCTCGCTAATTCACCTTA

8886 bin1, 8907 mbo11,

8941 GAAGCAAACAGACCTGGAAAAGTTCCCTTTCTGAGAGTAGCAACAGAAAGCTCTGCAAAG
CTTCGTTTGTCTGGACCTTTTCAAGGGAAAGACTCTCATCGTTGTCTTTCGAGACGTTTC

9001 ACTCCCTCCAAGCTATTGGATCCTCTTGCTTGGGATAACCACTATGGTACTCAGATACCA
TGAGGGAGGTTCGATAACCTAGGAGAACGAACCCTATTGGTGATACCATGAGTCTATGGT

9008 bstXI, 9018 bamh1 bin1,

9061 AAAGAAGAGTGGAAATCCCAAGAGAAGTCACCAGAAAAAACAGCTTTTAAGAAAAAGGAT
TTTCTTCTCACCTTTAGGGTTCTCTTCAGTGGTCTTTTTTGTCGAAAATTCTTTTTCCTA

9064 mbo11,

9121 ACC[?]TTTTGT[?]CCTGAACGCTTGTGAAAGCAATCATGCAATAGCAGCAATAAATGAGGGA
TGG[?]AAAACAGG[?]ACTTGCGAACACTTTCGTTAGTACGTTATCGTCGTTATTTACTCCCT

9181 CAAAATAAGCCCGAAATAGAAGTCACCTGGGCAAAGCAAGGTAGGACTGAAAGGCTGTGC
GTTTTATTCGGGCTTTATCTTCAGTGGACCCGTTTCGTTCCATCCTGACTTTCCGACACG

9241 TCTCAAAACCCACCAGTCTTGAAACGCCATCAACGGGAAATAACTCGTACTACTCTTCAG
AGAGTTTTGGGTGGTCAGAACTTTGCGGTAGTTGCCCTTTATTGAGCATGATGAGAAGTC

9294 mbo11,

9301 TCAGATCAAGAGGAAATTGACTATGATGATACCATATCAGTTGAAATGAAGAAGGAAGAT
AGTCTAGTTCTCCTTTAACTGATACTACTATGGTATAGTCAACTTTACTTCTTCCTTCTA

9348 mbo11, 9355 mbo11,

9361 TTTGACATTTATGATGAGGATGAAAATCAGAGCCCCCGCAGCTTTCAAAAGAAAACACGA
AAACTGTAAATACTACTCCTACTTTTAGTCTCGGGGGCGTCGAAAGTTTTCTTTTGTGCT

9421 CACTATTTTATTGCTGCAGTGGAGAGGCTCTGGGATTATGGGATGAGTAGCTCCCCACAT
GTGATAAAATAACGACGTCACCTCTCCGAGACCCTAATACCCTACTCATCGAGGGGTGTA

9434 pst1,

9481 GTTCTAAGAAACAGGTATGAATGCATTGGTTATTCCTTTGCTCTGCTCTTGTGACATTTG
CAAGATTCTTTGTCCATACTTACGTAACCAATAAGGAAACGAGACGAGAACACTGTAAAC

9501 ava3,

9541 ACTTTACCAGATGATGACACCAACAATGAGAGTTAGAGGATGAAAAATCAAGGTGTCTTG
TGAAATGGTCTACTACTGTGGTTGTTACTCTCAATCTCCTACTTTTTAGTTCCACAGAAC

9601 AAACTCTTACTCTGGTGAGATAGAGAGAAGAGAGGTTTACAAGAGGGTAGAGGAATCAGG
TTTGAGAATGAGACCACTCTATCTCTCTTCTCTCCAAATGTTCTCCCATCTCCTTAGTCC

9627 mbo11,

9661 GACAAGAAGGAGCAATGGGGAGCAGGAAGAAAGTCAACCTCACCTATCAACCTTATGAGC
CTGTTCTTCCTCGTTACCCCTCGTCCTTCTTTCAGTTGGAGTGGATAGTTGGAATACTCG

9686 mbo11, 9720 pst1,

9721 TGCAGGGGAAATCTTAGTCTCATCAGAGAACTTTAAGCAAGGCAGAAGGAAGAAGCAAGT
ACGTCCCCTTTAGAATCAGAGTAGTCTCTTGAAATTCGTTCCGTCTTCCTTCTTCGTTCA

9769 mbo11,

9781 TCGTGTTAAAGAGGTTAAGGACTTGGGGAGGATTGGTTTTCATGATACTGGGGATCCAGA
AGCACAATTTCTCCAATTCCTGAACCCCTCCTAACCAAAAGTACTATGACCCCTAGGTCT

9832 bamh1 binI,

9841 GGGCAAAGTAGGATGATTTTATTTTTAGGTGAGTAGAGGGAGGTTGAGCAGTCAAGTAGT
CCCGTTTCATCCTACTAAAATAAAAATCCACTCATCTCCCTCCAACTCGTCAGTTCATCA

9901 ACAGAGAAATGTGGAATATAAACATAAGGAGAGTCAGTCAGTCACAAATATTTATGGAAC
TGTCTCTTTACACCTTATATTTGTATTCCTCTCAGTCAGTCAGTGTTTATAAATACCTTG

9961 ACCTACTACATGCCAGACACTTTTGTACACATTGGAGTGCAGCACAGCAGGAGCTTATAT
TGGATGATGTACGGTCTGTGAAAACATGTGTAACCTCACGTCGTGTCGTCCTCGAATATA

0021 TCTATTGGAAAGAGCAAGAGACAAGTTAGTACAAAACTAAATAAGGCAATCTCAATTGTG
AGATAACCTTTCTCGTTCTCTGTTCAATCATGTTTTGATTTATTCCGTTAGAGTTAACAC

0081 TTGTTCAGAAGATAGAATAGAATAATATGGTAGAGAGAATAATATGATAGAGTAATACGT
AACAAGTCTTCTATCTTATCTTATTATACCATCTCTCTTATTATACTATCTCATTATGCA

10088 mbo11,

0141 AGAGTGTTACATTAGCTGTGGTGGTCATAGAAATCATCTTTGAGGAGTTGAGACCTAAAT
TCTCACAATGTAATCGACACCACCAGTATCTTTAGTAGAAACTCCTCAACTCTGGATTTA

0201 GATGATAACAGGCCAGCTGATGGAGCTCTGAGAGAAGATGTTTCCAGACAGAGGGAAGAG
CTACTATTGTCCGGTCGACTACCTCGAGACTCTCTTCTACAAAGGTCTGTCTCCCTTCTC

10214 pvu11, 10223 sac1, 10234 mbo11, 10255 mbo11,

0261 AAAATGCAAAGGCCCTGAGAATGGAAAAAGTTTACCATGTTTGAGGAACAAGAAGGGACA
TTTTACGTTTCCGGGACTCTTACCTTTTTCAAATGGTACAAACTCCTTGTTCTTCCCTGT

0321 AGCATGACTTGTCACTTTCCCACCTTGCTTTCTAGCTCAAGTAAGCCCTTATTGCTGCAT
TCGTACTGAACAGTGAAAGGGTGGAACGAAAGATCGAGTTCATTCGGGAATAACGACGTA

0381 TGCAACCATAGACTATTTCCCTAGTCCACTGACTTTCTCATTCTCCTGGACCTCTATCTT
ACGTTGGTATCTGATAAAGGGATCAGGTGACTGAAAGAGTAAGAGGACCTGGAGATAGAA

0441 AGATTTTCTGTGTTTTCCTTAAATCTCGAGCACCTTTTTTCCATTCTCCAATGCCCATTT
TCTAAAAGACACAAAAGGAATTTAGAGCTCGTGGAAAAAAGGTAAGAGGTTACGGGTAAA

10465 ava1 xho1,

0501 TCACTCTTAGCTGATGCTGTGCAATGAACCTCCACAGACTCCCAACATCACATTTACTGG
AGTGAGAATCGACTACGACACGTTACTTGGAGGTGTCTGAGGGTTGTAGTGTAAATGACC

0561 CATTTGTACCCATATACTCTGCCTGCACACTTATTACAATAAATAGAGTGTCTGTGCTCC
GTAAACATGGGTATATGAGACGGACGTGTGAATAATGTTATTTATCTCACAGACACGAGG

10619 mstII,

0621 TAAGGCCAACCTCTCCAGTTTTGTACTTGATTCCCATCTCTTCTCCCTTCATAAAAAATG
ATTCCGGTTGGAGAGGTCAAAACATGAACTAAGGGTAGAGAAGAGGGAAGTATTTTTTAC

10659 mbo11,

0681 TTGCTCCAGCAATTTCTCCTTTCTCCCTTACATCATCAATTTATCTCTCCACTAGATTAA
AACGAGGTCGTTAAAGAGGAAAGAGGGAATGTAGTAGTTAAATAGAGAGGTGATCTAATT

0741 TCTCATCAACATCAATCATGCTATTATTTCCTCCCCTATCTTTACAGAAACCGTCTTAAA
AGAGTAGTTGTAGTTAGTACGATAATAAAGGAGGGGATAGAAATGTCTTTGGCAGAATTT

0801 ATAATTGTCCCTGCTTGCAGCCTTTGCATCTTTTCCTGCCATTCTCTCCTCTTTAACCCA
TATTAACAGGGACGAACGTCGGAAACGTAGAAAAGGACGGTAAGAGAGGAGAAATTGGGT

0861 CTCCAAAGAAGCTTTTATCTCTATTATTTCATAAGAACTACATTTTCAAGGTCATTATTG
GAGGTTTCTTCGAAAATAGAGATAATAAAGTATTCTTGATGTAAAAGTTCCAGTAATAAC

10869 hind111,

0921 ATCTTTACATTACTGATTACTAGCCCTCATCTTATTTGAATTCTCAGTAGTATTTGAGAG EcoRI :#76
TAGAAATGTAATGACTAATGATCGGGAGTAGAATAAACTTAAGAGTCATCATAAACTCTC

10958 ecor1,

0981 GTGATTACTCCATCGTTGATACAGGTTCTCACTTGGTTTCTCTAACACCACTTTTCTTCA
CACTAATGAGGTAGCAACTATGTCCAAGAGTGAACCAAAGAGATTGTGGTGAAAAGAAGT

11035 mbo11,

1041 CTTTTCTACTAAACTTACTGCCAATCTTTGTCAACTTTGCGAGTCCTTCTTCATCCCCCT
GAAAAGATGATTTGAATGACGGTTAGAAACAGTTGAAACGCTCAGGAAGAAGTAGGGGGA

11088 mbo11,

1101 AATCTTTAAAGGTTAGAGTGCTCCAGGGATTAGTTCTCCAACTTCTTCTTTATTCTATTT
TTAGAAATTTCCAATCTCACGAGGTCCCTAATCAAGAGGTTGAAGAAGAAATAAGATAAA

11105 aha111, 11144 mbo11,

1161 ATACTCACTCTGTGACGACATTGTTTAGCCTCATGATTTTAAGTACTATCTATCTGCAGA
TATGAGTGAGACACTGCTGTAACAAATCGGAGTACTAAAATTCATGATAGATAGACGTCT

11202 sca1, 11214 pst1,

1221 TAATTCCAGTTGAATATCTGGAGATATTCTCAGATCTCTCTCCTGAAGTCAAAATTTACA
ATTAAGGTCAACTTATAGACCTCTATAAGAGTCTAGAGAGAGGACTTCAGTTTTAAATGT

11252 bgl11,

1281 TATCAACTCCCTACCTTAACATGCACACAATTAGACTCTTAATTCCTGCCCCTTCCCAAA
ATAGTTGAGGGATGGAATTGTACGTGTGTTAATCTGAGAATTAAGGACGGGGAAGGGTTT

1341 ACCTGCTGATTGCTCAGCCTTCTCCATGGCAATCAAAAACCATTCCTGGCCGGGCATGGT
TGGACGACTAACGAGTCGGAAGAGGTACCGTTAGTTTTTGGTAAGGACCGGCCCGTACCA

11364 nco1,

1401 GGCTCACTCCTGTAACCCCAGCACTTGGGGAAGCTGAGGCAGGTGGATTGGTTGAGCCCC
CCGAGTGAGGACATTGGGGTCGTGAACCCCTTCGACTCCGTCCACCTAACCAACTCGGGG

11434 tth1111,

1461 AGAGTTCAAGATCAGCCTGGGCAACATGGCAAAACCTCATCTCTATTAAAAATACAAAAA
TCTCAAGTTCTAGTCGGACCCGTTGTACCGTTTTGGAGTAGAGATAATTTTTATGTTTTT

1521 TTAGCCAGGCGTGGTGGTGCACACCTGCAGTCCCAGCTACTCAGGAGGCTGAGGCACGAG
AATCGGTCCGCACCACCACGTGTGGACGTCAGGGTCGATGAGTCCTCCGACTCCGTGCTC

11545 pst1,

1581 AATCGCTTGAACTGGGGAGACGGAGGTTGCAGTGAGCTGAGATGGCCTCACTGCACTCCA
TTAGCGAACTTGACCCCTCTGCCTCCAACGTCACTCGACTCTACCGGAGTGACGTGAGGT

1641 GCCTGGGCAACAGAGTCAGACTTGATCTCAAAAAAATAAATAAGTAAAACCATTCCCTAT
CGGACCCGTTGTCTCAGTCTGAACTAGAGTTTTTTTATTTATTCATTTTGGTAAGGGATA

1701 TTCAGTTATTCAGTCACAGATCTTGAAGTTTTCCAACTCTTTTTTTCTTACATTAACATC
AAGTCAATAAGTCAGTGTCTAGAACTTCAAAAGGTTGAGAAAAAAAGAATGTAATTGTAG

11718 bgl11,

1761 TAATCTGTAAAGGATCCTGTATTAGTCCATTTTCACACTGCTAATAAAGACATACCTGAG
ATTAGACATTTCCTAGGACATAATCAGGTAAAAGTGTGACGATTATTTCTGTATGGACTC

11772 bamh1 binI,

1821 ACTGGGCAATTTACAAAAGAAAGAAGTTTAATGGACTCACAGTTCCACATGGCTGGGGAG
TGACCCGTTAAATGTTTTCTTTCTTCAAATTACCTGAGTGTCAAGGTGTACCGACCCCTC

11865 bstXI, 11879 stu1,

1881 GCCTCACAATCATGGCAGAAGGCAGGGAGGAGCAAGTTATATCTCACATGATGGCACAGG
CGGAGTGTTAGTACCGTCTTCCGTCCCTCCTCGTTCAATATAGAGTGTACTACCGTGTCC

1941 CAAAGAGAGAGAGCTTGTGCAGGGAAACTCCTCTTTTTAAAACCATCAGATCTTGTGAGA
GTTTCTCTCTCTCGAACACGTCCCTTTGAGGAGAAAAATTTTGGTAGTCTAGAACACTCT

11976 aha111, 11988 bgl11,

2001 CTTATTCACTATCATGAGAACAGCAAGGGAAGGACCTACTGCCATGATTCAGTTACCTCC
GAATAAGTGATAGTACTCTTGTCGTTCCCTTCCTGGATGACGGTACTAAGTCAATGGAGG

2061 TATCAGGTCCCTCCCACAGAACATGGAATTCAAGATGAGATTTGGGTAGGGACACAGCC EcoRI #17
ATAGTCCAGGGAGGGTGTCTTGTACCTTAAGTTCTACTCTAAACCCATCCCTGTGTCGG

12087 ecor1,

2121 AAACCATATCATTCCACCCCATCCCCTCCCAAATCTCGTGTCCTCACATTTCAAAACCAA
TTTGGTATAGTAAGGTGGGGTAGGGGAGGGTTTAGAGCACAGGAGTGTAAAGTTTTGGTT

2181 TCATGCCTTCCCAACAGTCCCCCAAAGTCATAACACATTTCAGCATTAACTCAAAAGTCC
AGTACGGAAGGGTTGTCAGGGGGTTTCAGTATTGTGTAAAGTCGTAATTGAGTTTTCAGG

2241 ACAGTCCAATGTCTCATCTAAGACAAGGCAAGTCCCTTCCACCTATGAGCATGTAAAGTC
TGTCAGGTTACAGAGTAGATTCTGTTCCGTTCAGGGAAGGTGGATACTCGTACATTTCAG

2301 AAAAGCAAGTTAGTTACTTCCTAGATACAATGGGGTATAGGCATTGGGTAAATACAGCCA
TTTTCGTTCAATCAATGAAGGATCTATGTTACCCCATATCCGTAACCCATTTATGTCGGT

2361 TTCCAAATGGGAGAAATTGGCCAAAACAAAGGGCCTACAGGCCCCATGCAAGTCTGAAAT
AAGGTTTACCCTCTTTAACCGGTTTTGTTTCCCGGATGTCCGGGGTACGTTCAGACTTTA

12378 bal1,

2421 TCTACGGGGCAGTCAAATCTTAAATCTCTAAAGTGATCTCCTTTGACTCCATGTCTTGCA
AGATGCCCCGTCAGTTTAGAATTTAGAGATTTCACTAGAGGAAACTGAGGTACAGAACGT

2481 TCTGAGTCATGCTGATGCAAGAGGTGGGTGCCCATGGTCTTGGGCAGCTCCACCCCTGTG
AGACTCAGTACGACTACGTTCTCCACCCACGGGTACCAGAACCCGTCGAGGTGGGGACAC

12512 bstXI nco1, 12530 bstXI,

2541 GCTTTGCAGGGTACAGCCTCCCTTGTGTCTGCCTTCATGGGCTGGCATTTTCTGTAGCTT
CGAAACGTCCCATGTCGGAGGGAACACAGACGGAAGTACCCGACCGTAAAAGACATCGAA

2601 TTCCAGATGCTGGTGCAAGTTTCTGATGGATCTACCATTCCGGGGTCTGGAGGACGGTGG
AAGGTCTACGACCACGTTCAAAGACTACCTAGATGGTAAGGCCCCAGACCTCCTGCCACC

12628 binI,

2661 CC[?]TCTT[?]TCACAGCTCCACTAGGTGGTACCCCAGTAGGGACTCTGTGTGGGTGCCCTGA
GG[?]A[?]AAGA[?]TGT[?]GA[?][?]TGATCCACCATGGGGTCATCCCTGAGACACACCCACGGGACT

12[?]6[?] [?][?][?][?][?], 12685 kpn1,

2721 GCCCACATTTCCCTTCTGCACTGCCCTAGCAGAGTTTCTACATGAAGGCCCACCCCCGCA
CGGGTGTAAAGGGAAGACGTGACGGGATCGTCTCAAAGATGTACTTCCGGGTGGGGGCGT

2781 GCAAACTTCTGCCTGGGCATCCAGGCACTTCCATTAATTTCTAAACAACAAAGCATTCAA
CGTTTGAAGACGGACCCGTAGGTCCGTGAAGGTAATTAAAGATTTGTTGTTTCGTAAGTT

2841 GAGGCGACTTGGGTGCTATTAAGGGCATTCAGTTTCATAAGGGAAGCAGAGCATAAAAGT
CTCCGCTGAACCCACGATAATTCCCGTAAGTCAAAGTATTCCCTTCGTCTCGTATTTTCA

2901 TTGGAAAATTTGCAGCCTGATAATGTTAGAGAAAAGCAAATCCCATTTTCTGAGGAGAAA
AACCTTTTAAACGTCGGACTATTACAATCTCTTTTCGTTTAGGGTAAAAGACTCCTCTTT

2961 TTCAAGCAGGCTGCAGAAATTTGCATAAGTAATGAGGAGCCGAATGTTAATCCCAAGACA
AAGTTCGTCCGACGTCTTTAAACGTATTCATTACTCCTCGGCTTACAATTAGGGTTCTGT

12971 pst1, 13013 bstXI,

3021 ATGGGGAAAATGTCTCCAGGGCATGTCAGAGGTCTTCACACCAGCCCCTCCCATCACCGG
TACCCCTTTTACAGAGGTCCCGTACAGTCTCCAGAAGTGTGGTCGGGGAGGGTAGTGGCC

13053 mbo11,

3081 CCCAGAGGCCTAAGAGGAAAAAGTTGTTTCATGGGCTGGGCCCAGGATCCCCATGCTGTG
GGGTCTCCGGATTCTCCTTTTTCAACAAAGTACCCGACCCGGGTCCTAGGGGTACGACAC

13086 stu1, 13118 apa1, 13125 bamh1 binI,

3141 TGCAGCCTAGGGAGTCGGTGCCTTGTGTCCCAGCCGCTACAGCCATGGCTGAAAGGGGTC
ACGTCGGATCCCTCAGCCACGGAACACAGGGTCGGCGATGTCGGTACCGACTTTCCCCAG

13146 avr2, 13183 nco1,

3201 AATGTAGAGCTCAGGCTGTGGCTTCATAGGGTACAAGTCCCAAGCCTTGGCAGCTTCCAC
TTACATCTCGAGTCCGACACCGAAGTATCCCATGTTCAGGGTTCGGAACCGTCGAAGGTG

13207 sac1,

3261 ATGATGTTGAGCCTGCGAGTGCACAGAAGTCAAGAACTGGGGTTTGGGAACTTCCGCCTA
TACTACAACTCGGACGCTCACGTGTCTTCAGTTCTTGACCCCAAACCCTTGAAGGCGGAT

3321 GATTTCAGAAGATGTATGAAAACACCTGGATGCCTAGGCAGAAGTTTGCTGCGGGGGTGG
CTAAAGTCTTCTACATACTTTTGTGGACCTACGGATCCGTCTTCAAACGACGCCCCCACC

13328 mbo11, 13353 avr2, 13379 apa1,

3381 GCCCTCATGGAGAACCTCTGCTAGGGCAGTGCAGAAGGGAAATGTGGGCTCAGAGCACAC
CGGGAGTACCTCTTGGAGACGATCCCGTCACGTCTTCCCTTTACACCCGAGTCTCGTGTG

3441 ACACAGAGTCCCTGCTGGGGTACCACCTAGTGGAGCTGTGAGAAGAGGACCACCGTCCTC
TGTGTCTCAGGGACGACCCCATGGTGGATCACCTCGACACTCTTCTCCTGGTGGCAGGAG

13459 kpn1, 13482 mbo11,

3501 CAGGCCCCAGAATGGTAGATACCCTAAATCATCTCTTTCAAATTCGAAGTTCCACAAATC
GTCCGGGGTCTTACCATCTATGGGATTTAGTAGAGAAAGTTTAAGCTTCAAGGTGTTTAG

.3561 TCTAGGACAGGAGCAAAATGCCACCAGTATCTTTGCTAAAACATAACAAGAGTTACCTTT
AGATCCTGTCCTCGTTTTACGGTGGTCATAGAAACGATTTTGTATTGTTCTCAATGGAAA

3621 GCTCCAGTTCCCAACAAGTTCCTCATCTCTATCTGAGACCACCTCAGCCTGGATTTCATT
CGAGGTCAAGGGTTGTTCAAGGAGTAGAGATAGACTCTGGTGGAGTCGGACCTAAAGTAA

3681 GTCCATATCATTATCAGCCTTTTGGTCAAAGCCATTCAACAAGTCTCTAGAGAGTTCCAA
CAGGTATAGTAATAGTCGGAAAACCAGTTTCGGTAAGTTGTTCAGAGATCTCTCAAGGTT

13726 xba1,

3741 ACTTTCCACATTTTCCTGTCGTCTTCTGAGCCCTCCAAACTGTTCCAACCTCTGCCTGTT
TGAAAGGTGTAAAAGGACAGCAGAAGACTCGGGAGGTTTGACAAGGTTGGAGACGGACAA

13762 mbo11,

.3801 ACCCAGTTCCAAAGTCGCTTCCACATTTTTGGGTATCTTTTCAGCAGCACCCCACTCTAC
TGGGTCAAGGTTTCAGCGAAGGTGTAAAAACCCATAGAAAAGTCGTCGTGGGGTGAGATG

13821 bstXI, 13852 bstXI,

.3841 TG[?]TAC[?]AACTTACTGTATTAGTCTGTTCTCACACTGCTGATAAAGATATACCTGAGACT
AC[?][?]TG[?][?]TTGAATGACATAATCAGAC[?]AGAGTGTGACGACTATTTCTATATGGACTCTGA

1[?][?][?][?] [?][?][?]1,

13921 GGGAAATTTACAAAGGAAAGAGGTTTTATGGACTTACTGTTCCACATGGCTGGGGAGGCC
CCCTTTAAATGTTTCCTTTCTCCAAAATACCTGAATGACAAGGTGTACCGACCCCTCCGG

13962 bstXI, 13976 stu1,

13981 TCAAAATCATGGCAGAAGGCAAGGAGGAGCAAGTCTTGTCTTACATGGATGGCAGCAGGC
AGTTTTAGTACCGTCTTCCGTTCCTCCTCGTTCAGAACAGAATGTACCTACCGTCGTCCG

14041 AAAGAAAGAAAGCTTGTGCAGGAAAACTCTTTTTTTTCTTTTCTTCTTTTTAGACAGAGA
TTTCTTTCTTTCGAACACGTCCTTTTGAGAAAAAAAAGAAAAGAAGAAAAATCTGTCTCT

14050 hind111, 14082 mbo11, 14098 bgl11,

14101 TCTTGCTCTGTCACGCAGGCTGGAGTCAGTGGCGTGAGTCTTGGCTCACTGCAACCTCCA
AGAACGAGACAGTGCGTCCGACCTCAGTCACCGCACTCAGAACCGAGTGACGTTGGAGGT

14161 CCTCCCGGGTTCAAGTGATTCTCCTGCCTCAGCCTTCCAAGTAGCTGGTACTACAGGCAT
GGAGGGCCCAAGTTCACTAAGAGGACGGAGTCGGAAGGTTCATCGACCATGATGTCCGTA

14164 ava1 sma1 xma1, 14184 tthIII1, 14197 bstXI,

14221 GTGCCACCACATGAGATGAGATTTTGGTAGGGACACAGCCAAACCATATCATTCCCCCTG
CACGGTGGTGTACTCTACTCTAAAACCATCCCTGTGTCGGTTTGGTATAGTAAGGGGGAC

14281 TCCCCTCCCAAATCTCATGTCCTCACATTTCAAAACCAATCATGCCTTCCCAACAGTCCC
AGGGGAGGGTTTAGAGTACAGGAGTGTAAAGTTTTGGTTAGTACGGAAGGGTTGTCAGGG

14341 CCAAAGTCATAACTCATTTCAGCATTAACTCAAAAGTCCACAGTATTTTTAGTAGAGATG
GGTTTCAGTATTGAGTAAAGTCGTAATTGAGTTTTCAGGTGTCATAAAAATCATCTCTAC

14401 GGGTTTCACCATATTGGCCAGACTGGTCTCTAACTCCTGACCTTATGATCCGCCCGCCTT
CCCAAAGTGGTATAACCGGTCTGACCAGAGATTGAGGACTGGAATACTAGGCGGGCGGAA

14415 bal1,

14461 GGCCTCCCAAAGCACTGGGATTACAGGTGTGAGCCACCATGCCCAGCCAAAAACTCTTCT
CCGGAGGGTTTCGTGACCCTAATGTCCACACTCGGTGGTACGGGTCGGTTTTTGAGAAGA

14467 bstXI, 14515 mbo11,

14521 TTTTAAAACCATCAGTTCTCGATGAGACTTATTCACTATCACAAGAACAGCATAGGAAAG
AAAATTTTGGTAGTCAAGAGCTACTCTGAATAAGTGATAGTGTTCTTGTCGTATCCTTTC

14522 aha111, 14579 bgl11,

14581 ATCTGCCCCCATGATTCAATTACCTCCCACCAGGTCCCTCCCACAACACATGGGAATTCA EcoRI #55
TAGACGGGGGTACTAAGTTAATGGAGGGTGGTCCAGGGAGGGTGTTGTGTACCCTTAAGT

14634 ecor1,

4641 CGATGAGATTTGGGTGGGGATCCTCTTGGCTATCATTTCAAAAAATAGTCAGGGTTGATT
GCTACTCTAAACCCACCCCTAGGAGAACCGATAGTAAAGTTTTTTATCAGTCCCAACTAA

14658 bamh1 bin1,

.4701 ATGGACTTAGTATTGAATAAGATGAAAATGTACTGAAATGGAACGATGAAGATAATTGAC
TACCTGAATCATAACTTATTCTACTTTTACATGACTTTACCTTGCTACTTCTATTAACTG

14748 mbo11,

4761 CAGGGAAAGCAGGTTACAAGCCAAGAGAGGATACAGTGTCACTTCAGATGCTATAAAAGT
GTCCCTTTCGTCCAATGTTCGGTTCTCTCCTATGTCACAGTGAAGTCTACGATATTTTCA

4821 GATGTTTTTATTTTTTGAATGTTATTTGGCTATATTTTCAAATTATCTGCACTGCATATG
CTACAAAAATAAAAAACTTACAATAAACCGATATAAAAGTTTAATAGACGTGACGTATAC

14875 nde1,

4881 CACTGCTTCTGTAATAATAAAAGGGTATTAAAATAATAAAATCAAGTCTATAAATCACAC
GTGACGAAGACATTATTATTTTCCCATAATTTTATTATTTTAGTTCAGATATTTAGTGTG

4941 ACACACACCCCAGCCTCTTCTCTACACCTCAGTGACTACCAACCACATCTAAATTATCAT
TGTGTGTGGGGTCGGAGAAGAGATGTGGAGTCACTGATGGTTGGTGTAGATTTAATAGTA

14956 mbo11,

.5001 CATTTCTCATCTGAATTATTGCAAGAAGCCATTGCAATAATTCAGATGAGAGTTGATACA
GTAAAGAGTAGACTTAATAACGTTCTTCGGTAACGTTATTAAGTCTACTCTCAACTATGT

:5061 AAGCCTTCCTGTTTCCAACCTTACCCTCATATAGTCTTTTCCCAACATTGCATCTAAAGT
TTCGGAAGGACAAAGGTTGGAATGGGAGTATATCAGAAAAGGGTTGTAACGTAGATTTCA

.5121 AATTTTTCAAATTGCAATTCTGATCCGGGGAATTC
TTAAAAAGTTTAACGTTAAGACTAGGCCCCTTAAG

15150 ecor1,

APPENDIX B

ValTyrGlyPheTrpGlyAlaThrThrGlnThrPheGlySerArgGlyMetThrAlaLeu
1 GTCTATGGATTCTGGGGTGCCACAACTCAGACTTTCGGAAGCAGAGGCATGACCGCCTTA
CAGATACCTAAGACCCCACGGTGTTGAGTCTGAAAGCCTTCGTCTCCGTACTGGCGGAAT

LeuLysValSerSerCysAspLysAsnThrGlyAspTyrTyrGluAspSerTyrGluAsp
61 CTGAAGGTTTCTAGTTGTGACAAGAACACTGGTGATTATTACGAGGACAGTTATGAAGAT
GACTTCCAAAGATCAACACTGTTCTTGTGACCACTAATAATGCTCCTGTCAATACTTCTA

115 mbo11,

IleSerAlaTyrLeuLeuSerLysAsnAsnAlaIleGluProArgSerPheSerGlnAsn
121 ATTTCAGCATACTTGCTGAGTAAAAACAATGCCATTGAACCAAGAAGCTTCTCCCAGAAT
TAAAGTCGTATGAACGACTCATTTTTGTTACGGTAACTTGGTTCTTCGAAGAGGGTCTTA

165 hind111, 177 ecor1,

SerArgHisProSerThrArgGlnLysGlnPheAsnAlaThrThrIleProGluAsnAsp
181 TCAAGACACCCTAGCACTAGGCAAAAGCAATTTAATGCCACCACAATTCCAGAAAATGAC
AGTTCTGTGGGATCGTGATCCGTTTTCGTTAAATTACGGTGGTGTTAAGGTCTTTTACTG

IleGluLysThrAspProTrpPheAlaHisArgThrProMetProLysIleGlnAsnVal
241 ATAGAGAAGACTGACCCTTGGTTTGCACACAGAACACCTATGCCTAAAATACAAAATGTC
TATCTCTTCTGACTGGGAACCAAACGTGTGTCTTGTGGATACGGATTTTATGTTTTACAG

246 mbo11,

SerSerSerAspLeuLeuMetLeuLeuArgGlnSerProThrProHisGlyLeuSerLeu
301 TCCTCTAGTGATTTGTTGATGCTCTTGCGACAGAGTCCTACTCCACATGGGCTATCCTTA
AGGAGATCACTAAACAACTACGAGAACGCTGTCTCAGGATGAGGTGTACCCGATAGGAAT

329 tthIII1,

SerAspLeuGlnGluAlaLysTyrGluThrPheSerAspAspProSerProGlyAlaIle
361 TCTGATCTCCAAGAAGCCAAATATGAGACTTTTTCTGATGATCCATCACCTGGAGCAATA
AGACTAGAGGTTCTTCGGTTTATACTCTGAAAAAGACTACTAGGTAGTGGACCTCGTTAT

AspSerAsnAsnSerLeuSerGluMetThrHisPheArgProGlnLeuHisHisSerGly
421 GACAGTAATAACAGCCTGTCTGAAATGACACACTTCAGGCCACAGCTCCATCACAGTGGG
CTGTCATTATTGTCGGACAGACTTTACTGTGTGAAGTCCGGTGTCGAGGTAGTGTCACCC

468 bstXI,

AspMetValPheThrProGluSerGlyLeuGlnLeuArgLeuAsnGluLysLeuGlyThr
481 GACATGGTATTTACCCCTGAGTCAGGCCTCCAATTAAGATTAAATGAGAAACTGGGGACA
CTGTACCATAAATGGGGACTCAGTCCGGAGGTTAATTCTAATTTACTCTTTGACCCCTGT

497 tthIII1, 504 stu1,

ThrAlaAlaThrGluLeuLysLysLeuAspPheLysValSerSerThrSerAsnAsnLeu
541 ACTGCAGCAACAGAGTTGAAGAAACTTGATTTCAAAGTTTCTAGTACATCAAATAATCTG
TGACGTCGTTGTCTCAACTTCTTTGAACTAAAGTTTCAAAGATCATGTAGTTTATTAGAC

542 pst1, 558 mbo11,

IleSerThrIleProSerAspAsnLeuAlaAlaGlyThrAspAsnThrSerSerLeuGly
601 ATTTCAACAATTCCATCAGACAATTTGGCAGCAGGTACTGATAATACAAGTTCCTTAGGA
TAAAGTTGTTAAGGTAGTCTGTTAAACCGTCGTCCATGACTATTATGTTCAAGGAATCCT

653 mstII,

ProProSerMetProValHisTyrAspSerGlnLeuAspThrThrLeuPheGlyLysLys
661 CCCCCAAGTATGCCAGTTCATTATGATAGTCAATTAGATACCACTCTATTTGGCAAAAAG
GGGGGTTCATACGGTCAAGTAATACTATCAGTTAATCTATGGTGAGATAAACCGTTTTTC

SerSerProLeuThrGluSerGlyGlyProLeuSerLeuSerGluGluAsnAsnAspSer
721 TCATCTCCCCTTACTGAGTCTGGTGGACCTCTGAGCTTGAGTGAAGAAAATAATGATTCA
AGTAGAGGGGAATGACTCAGACCACCTGGAGACTCGAACTCACTTCTTTTATTACTAAGT

763 mbo11,

LysLeuLeuGluSerGlyLeuMetAsnSerGlnGluSerSerTrpGlyLysAsnValSer
781 AAGTTGTTAGAATCAGGTTTAATGAATAGCCAAGAAAGTTCATGGGGAAAAAATGTATCG
TTCAACAATCTTAGTCCAAATTACTTATCGGTTCTTTCAAGTACCCCTTTTTTACATAGC

SerThrGluSerGlyArgLeuPheLysGlyLysArgAlaHisGlyProAlaLeuLeuThr
841 TCAACAGAGAGTGGTAGGTTATTTAAAGGGAAAAGAGCTCATGGACCTGCTTTGTTGACT
AGTTGTCTCTCACCATCCAATAAATTTCCCTTTTCTCGAGTACCTGGACGAAACAACTGA

862 aha111, 875 sac1,

LysAspAsnAlaLeuPheLysValSerIleSerLeuLeuLysThrAsnLysThrSerAsn
901 AAAGATAATGCCTTATTCAAAGTTAGCATCTCTTTGTTAAAGACAAACAAAACTTCCAAT
TTTCTATTACGGAATAAGTTTCAATCGTAGAGAAACAATTTCTGTTTGTTTTGAAGGTTA

AsnSerAlaThrAsnArgLysThrHisIleAspGlyProSerLeuLeuIleGluAsnSer
961 AATTCAGCAACTAATAGAAAGACTCACATTGATGGCCCATCATTATTAATTGAGAATAGT
TTAAGTCGTTGATTATCTTTCTGAGTGTAACTACCGGGTAGTAATAATTAACTCTTATCA

ProSerValTrpGlnAsnIleLeuGluSerAspThrGluPheLysLysValThrProLeu
1021 CCATCAGTCTGGCAAAATATATTAGAAAGTGACACTGAGTTTAAAAAAGTGACACCTTTG
GGTAGTCAGACCGTTTTATATAATCTTTCACTGTGACTCAAATTTTTTCACTGTGGAAAC

1021 bstXI, 1060 aha111,

IleHisAspArgMetLeuMetAspLysAsnAlaThrAlaLeuArgLeuAsnHisMetSer
1081 ATTCATGACAGAATGCTTATGGACAAAAATGCTACAGCTTTGAGGCTAAATCATATGTCA
TAAGTACTGTCTTACGAATACCTGTTTTTACGATGTCGAAACTCCGATTTAGTATACAGT

1132 nde1,

AsnLysThrThrSerSerLysAsnMetGluMetValGlnGlnLysLysGluGlyProIle
1141 AATAAAACTACTTCATCAAAAAACATGGAAATGGTCCAACAGAAAAAAGAGGGCCCCATT
TTATTTTGATGAAGTAGTTTTTTGTACCTTTACCAGGTTGTCTTTTTTCTCCCGGGGTAA

1191 apa1,

ProProAspAlaGlnAsnProAspMetSerPhePheLysMetLeuPheLeuProGluSer
1201 CCACCAGATGCACAAAATCCAGATATGTCGTTCTTTAAGATGCTATTCTTGCCAGAATCA
GGTGGTCTACGTGTTTTAGGTCTATACAGCAAGAAATTCTACGATAAGAACGGTCTTAGT

AlaArgTrpIleGlnArgThrHisGlyLysAsnSerLeuAsnSerGlyGlnGlyProSer
1261 GCAAGGTGGATACAAAGGACTCATGGAAAGAACTCTCTGAACTCTGGGCAAGGCCCCAGT
CGTTCCACCTATGTTTCCTGAGTACCTTTCTTGAGAGACTTGAGACCCGTTCCGGGGTCA

cDNA clone
C1

ProLysGlnLeuValSerLeuGlyProGluLysSerValGluGlyGlnAsnPheLeuSer
1321 CCAAAGCAATTAGTATCCTTAGGACCAGAAAAATCTGTGGAAGGTCAGAATTTCTTGTCT
GGTTTCGTTAATCATAGGAATCCTGGTCTTTTTAGACACCTTCCAGTCTTAAAGAACAGA

1337 mstII,

GluLysAsnLysValValValGlyLysGlyGluPheThrLysAspValGlyLeuLysGlu
1381 GAGAAAAACAAAGTGGTAGTAGGAAAGGGTGAATTTACAAAGGACGTAGGACTCAAAGAG
CTCTTTTTGTTTCACCATCATCCTTTCCCACTTAAATGTTTCCTGCATCCTGAGTTTCTC

MetValPheProSerSerArgAsnLeuPheLeuThrAsnLeuAspAsnLeuHisGluAsn
1441 ATGGTTTTTCCAAGCAGCAGAAACCTATTTCTTACTAACTTGGATAATTTACATGAAAAT
TACCAAAAAGGTTCGTCGTCTTTGGATAAAGAATGATTGAACCTATTAAATGTACTTTTA

AsnThrHisAsnGlnGluLysLysIleGlnGluGluIleGluLysLysGluThrLeuIle
1501 AATACACACAATCAAGAAAAAAAAATTCAGGAAGAAATAGAAAAGAAGGAAACATTAATC
TTATGTGTGTTAGTTCTTTTTTTTTAAGTCCTTCTTTATCTTTTCTTCCTTTGTAATTAG

1531 mbo11,

GlnGluAsnValValLeuProGlnIleHisThrValThrGlyThrLysAsnPheMetLys
1561 CAAGAGAATGTAGTTTTGCCTCAGATACATACAGTGACTGGCACTAAGAATTTCATGAAG
GTTCTCTTACATCAAAACGGAGTCTATGTATGTCACTGACCGTGATTCTTAAAGTACTTC

1617 mbo11, 1620 xmn1,

AsnLeuPheLeuLeuSerThrArgGlnAsnValGluGlySerTyrAspGlyAlaTyrAla
1621 AACCTTTTCTTACTGAGCACTAGGCAAAATGTAGAAGGTTCATATGACGGGGCATATGCT
TTGGAAAAGAATGACTCGTGATCCGTTTTACATCTTCCAAGTATACTGCCCCGTATACGA

1661 nde1, 1673 nde1,

ProValLeuGlnAspPheArgSerLeuAsnAspSerThrAsnArgThrLysLysHisThr
1681 CCAGTACTTCAAGATTTTAGGTCATTAAATGATTCAACAAATAGAACAAAGAAACACACA
GGTCATGAAGTTCTAAAATCCAGTAATTTACTAAGTTGTTTATCTTGTTTCTTTGTGTGT

1683 sca1,

AlaHisPheSerLysLysGlyGluGluGluAsnLeuGluGlyLeuGlyAsnGlnThrLys
1741 GCTCATTTCTCAAAAAAAGGGGAGGAAGAAAACTTGGAAGGCTTGGGAAATCAAACCAAG
CGAGTAAAGAGTTTTTTTCCCCTCCTTCTTTTGAACCTTCCGAACCCTTTAGTTTGGTTC

1765 mbo11,

GlnIleValGluLysTyrAlaCysThrThrArgIleSerProAsnThrSerGlnGlnAsn
1801 CAAATTGTAGAGAAATATGCATGCACCACAAGGATATCTCCTAATACAAGCCAGCAGAAT
GTTTAACATCTCTTTATACGTACGTGGTGTTCCTATAGAGGATTATGTTCGGTCGTCTTA

1817 ava3, 1819 sph1, 1833 ecor5,

PheValThrGlnArgSerLysArgAlaLeuLysGlnPheArgLeuProLeuGluGluThr
1861 TTTGTCACGCAACGTAGTAAGAGAGCTTTGAAACAATTCAGACTCCCACTAGAAGAAACA
AAACAGTGCGTTGCATCATTCTCTCGAAACTTTGTTAAGTCTGAGGGTGATCTTCTTTGT

1890 xmn1, 1912 mbo11,

GluLeuGluLysArgIleIleValAspAspThrSerThrGlnTrpSerLysAsnMetLys
1921 GAACTTGAAAAAAGGATAATTGTGGATGACACCTCAACCCAGTGGTCCAAAAACATGAAA
CTTGAACTTTTTTCCTATTAACACCTACTGTGGAGTTGGGTCACCAGGTTTTTGTACTTT

HisLeuThrProSerThrLeuThrGlnIleAspTyrAsnGluLysGluLysGlyAlaIle
1981 CATTTGACCCCGAGCACCCTCACACAGATAGACTACAATGAGAAGGAGAAAGGGGCCATT
GTAAACTGGGGCTCGTGGGAGTGTGTCTATCTGATGTTACTCTTCCTCTTTCCCCGGTAA

1989 ava1,

ThrGlnSerProLeuSerAspCysLeuThrArgSerHisSerIleProGlnAlaAsnArg
2041 ACTCAGTCTCCCTTATCAGATTGCCTTACGAGGAGTCATAGCATCCCTCAAGCAAATAGA
TGAGTCAGAGGGAATAGTCTAACGGAATGCTCCTCAGTATCGTAGGGAGTTCGTTTATCT

2098 bgl11,

SerProLeuProIleAlaLysValSerSerPheProSerIleArgProIleTyrLeuThr
2101 TCTCCATTACCCATTGCAAAGGTATCATCATTTCCATCTATTAGACCTATATATCTGACC
AGAGGTAATGGGTAACGTTTCCATAGTAGTAAAGGTAGATAATCTGGATATATAGACTGG

ArgValLeuPheGlnAspAsnSerSerHisLeuProAlaAlaSerTyrArgLysLysAsp
2161 AGGGTCCTATTCCAAGACAACTCTTCTCATCTTCCAGCAGCATCTTATAGAAAGAAAGAT
TCCCAGGATAAGGTTCTGTTGAGAAGAGTAGAAGGTCGTCGTAGAATATCTTTCTTTCTA

2182 mbo11, 2190 mbo11,

SerGlyValGlnGluSerSerHisPheLeuGlnGlyAlaLysLysAsnAsnLeuSerLeu
2221 TCTGGGGTCCAAGAAAGCAGTCATTTCTTACAAGGAGCCAAAAAAAATAACCTTTCTTTA
AGACCCCAGGTTCTTTCGTCAGTAAAGAATGTTCCTCGGTTTTTTTTATTGGAAAGAAAT

AlaIleLeuThrLeuGluMetThrGlyAspGlnArgGluValGlySerLeuGlyThrSer
2281 GCCATTCTAACCTTGGAGATGACTGGTGATCAAAGAGAGGTTGGCTCCCTGGGGACAAGT
CGGTAAGATTGGAACCTCTACTGACCACTAGTTTCTCTCCAACCGAGGGACCCCTGTTCA

2307 bcl1,

AlaThrAsnSerValThrTyrLysLysValGluAsnThrValLeuProLysProAspLeu
2341 GCCACAAATTCAGTCACATACAAGAAAGTTGAGAACACTGTTCTCCCGAAACCAGACTTG
CGGTGTTTAAGTCAGTGTATGTTCTTTCAACTCTTGTGACAAGAGGGCTTTGGTCTGAAC

ProLysThrSerGlyLysValGluLeuLeuProLysValHisIleTyrGlnLysAspLeu
2401 CCCAAAACATCTGGCAAAGTTGAATTGCTTCCAAAAGTTCACATTTATCAGAAGGACCTA
GGGTTTTGTAGACCGTTTCAACTTAACGAAGGTTTTCAAGTGTAAATAGTCTTCCTGGAT

2422 xmn1,

PheProThrGluThrSerAsnGlySerProGlyHisLeuAspLeuValGluGlySerLeu
2461 TTCCCTACGGAAACTAGCAATGGGTCTCCTGGCCATCTGGATCTCGTGGAAGGGAGCCTT
AAGGGATGCCTTTGATCGTTACCCAGAGGACCGGTAGACCTAGAGCACCTTCCCTCGGAA

2490 bal1, 2499 binI, 2520 mbo11,

LeuGlnGlyThrGluGlyAlaIleLysTrpAsnGluAlaAsnArgProGlyLysValPro
2521 CTTCAGGGAACAGAGGGAGCGATTAAGTGGAATGAAGCAAACAGACCTGGAAAAGTTCCC
GAAGTCCCTTGTCTCCCTCGCTAATTCACCTTACTTCGTTTGTCTGGACCTTTTCAAGGG

PheLeuArgValAlaThrGluSerSerAlaLysThrProSerLysLeuLeuAspProLeu
2581 TTTCTGAGAGTAGCAACAGAAAGCTCTGCAAAGACTCCCTCCAAGCTATTGGATCCTCTT
AAAGACTCTCATCGTTGTCTTTCGAGACGTTTCTGAGGGAGGTTCGATAACCTAGGAGAA

2621 bstXI, 2631 bamh1 binI,

AlaTrpAspAsnHisTyrGlyThrGlnIleProLysGluGluTrpLysSerGlnGluLys
2641 GCTTGGGATAACCACTATGGTACTCAGATACCAAAAGAAGAGTGGAAATCCCAAGAGAAG
CGAACCCTATTGGTGATACCATGAGTCTATGGTTTTCTTCTCACCTTTAGGGTTCTCTTC

2677 mbo11,

cDNA clone
2-11

SerProGluLysThrAlaPheLysLysLysAspThrIleLeuSerLeuAsnAlaCysGlu
2701 TCACCAGAAAAAACAGCTTTTAAGAAAAAGGATACCATTTTGTCCCTGAACGCTTGTGAA
AGTGGTCTTTTTTGTCGAAAATTCTTTTTCCTATGGTAAAACAGGGACTTGCGAACACTT

SerAsnHisAlaIleAlaAlaIleAsnGluGlyGlnAsnLysProGluIleGluValThr
2761 AGCAATCATGCAATAGCAGCAATAAATGAGGGACAAAATAAGCCCGAAATAGAAGTCACC
TCGTTAGTACGTTATCGTCGTTATTTACTCCCTGTTTTATTCGGGCTTTATCTTCAGTGG

TrpAlaLysGlnGlyArgThrGluArgLeuCysSerGlnAsnProProValLeuLysArg
2821 TGGGCAAAGCAAGGTAGGACTGAAAGGCTGTGCTCTCAAAACCCACCAGTCTTGAAACGC
ACCCGTTTCGTTCCATCCTGACTTTCCGACACGAGAGTTTTGGGTGGTCAGAACTTTGCG
N-termini 80/77Kd

HisGlnArgGluIleThrArgThrThrLeuGlnSerAspGlnGluGluIleAspTyrAsp
2881 CATCAACGGGAAATAACTCGTACTACTCTTCAGTCAGATCAAGAGGAAATTGACTATGAT
GTAGTTGCCCTTTATTGAGCATGATGAGAAGTCAGTCTAGTTCTCCTTTAACTGATACTA

2907 mbo11,

AspThrIleSerValGluMetLysLysGluAspPheAspIleTyrAspGluAspGluAsn
2941 GATACCATATCAGTTGAAATGAAGAAGGAAGATTTTGACATTTATGATGAGGATGAAAAT
CTATGGTATAGTCAACTTTACTTCTTCCTTCTAAAACTGTAAATACTACTCCTACTTTTA

2961 mbo11, 2968 mbo11
N-termini 70/67Kd

GlnSerProArgSerPheGlnLysLysThrArgHisTyrPheIleAlaAlaValGluArg
3001 CAGAGCCCCCGCAGCTTTCAAAAGAAAACACGACACTATTTTATTGCTGCAGTGGAGAGG
GTCTCGGGGGCGTCGAAAGTTTTCTTTTGTGCTGTGATAAAATAACGACGTCACCTCTCC

3047 pst1,

LeuTrpAspTyrGlyMetSerSerSerProHisValLeuArgAsnArgAlaGlnSerGly
3061 CTCTGGGATTATGGGATGAGTAGCTCCCCACATGTTCTAAGAAACAGGGCTCAGAGTGGC
GAGACCCTAATACCCTACTCATCGAGGGGTGTACAAGATTCTTTGTCCCGAGTCTCACCG

SerValProGlnPheLysLysValValPheGlnGluPheThrAspGlySerPheThrGln
3121 AGTGTCCCTCAGTTCAAGAAAGTTGTTTTCCAGGAATTTACTGATGGCTCCTTTACTCAG
TCACAGGGAGTCAAGTTCTTTCAACAAAAGGTCCTTAAATGACTACCGAGGAAATGAGTC

ProLeuTyrArgGlyGluLeuAsnGluHisLeuGlyLeuLeuGlyProTyrIleArgAla
3181 CCCTTATACCGTGGAGAACTAAATGAACATTTGGGACTCCTGGGGCCATATATAAGAGCA
GGGAATATGGCACCTCTTGATTTACTTGTAAACCCTGAGGACCCCGGTATATATTCTCGT

GluValGluAspAsnIleMetValThrPheArgAsnGlnAlaSerArgProTyrSerPhe
3241 GAAGTTGAAGATAATATCATGGTAACTTTCAGAAATCAGGCCTCTCGTCCCTATTCCTTC
CTTCAACTTCTATTATAGTACCATTGAAAGTCTTTAGTCCGGAGAGCAGGGATAAGGAAG

3247 mbo11, 3278 stu1,

TyrSerSerLeuIleSerTyrGluGluAspGlnArgGlnGlyAlaGluProArgLysAsn
3301 TATTCTAGCCTTATTTCTTATGAGGAAGATCAGAGGCAAGGAGCAGAACCTAGAAAAAAC
ATAAGATCGGAATAAAGAATACTCCTTCTAGTCTCCGTTCCTCGTCTTGGATCTTTTTTG

3325 mbo11,

PheValLysProAsnGluThrLysThrTyrPheTrpLysValGlnHisHisMetAlaPro
3361 TTTGTCAAGCCTAATGAAACCAAAACTTACTTTTGGAAAGTGCAACATCATATGGCACCC
AAACAGTTCGGATTACTTTGGTTTTGAATGAAAACCTTTCACGTTGTAGTATACCGTGGG

3409 nde1,

ThrLysAspGluPheAspCysLysAlaTrpAlaTyrPheSerAspValAspLeuGluLys
3421 ACTAAAGATGAGTTTGACTGCAAAGCCTGGGCTTATTTCTCTGATGTTGACCTGGAAAAA
TGATTTCTACTCAAACTGACGTTTCGGACCCGAATAAAGAGACTACAACTGGACCTTTTT

AspValHisSerGlyLeuIleGlyProLeuLeuValCysHisThrAsnThrLeuAsnPro
3481 GATGTGCACTCAGGCCTGATTGGACCCCTTCTGGTCTGCCACACTAACACACTGAACCCT
CTACACGTGAGTCCGGACTAACCTGGGGAAGACCAGACGGTGTGATTGTGTGACTTGGGA

3492 stu1,

AlaHisGlyArgGlnValThrValGlnGluPheAlaLeuPhePheThrIlePheAspGlu
3541 GCTCATGGGAGACAAGTGACAGTACAGGAATTTGCTCTGTTTTTCACCATCTTTGATGAG
CGAGTACCCTCTGTTCACTGTCATGTCCTTAAACGAGACAAAAAGTGGTAGAAACTACTC

ThrLysSerTrpTyrPheThrGluAsnMetGluArgAsnCysArgAlaProCysAsnIle
3601 ACCAAAAGCTGGTACTTCACTGAAAATATGGAAAGAAACTGCAGGGCTCCCTGCAATATC
TGGTTTTCGACCATGAAGTGACTTTTATACCTTTCTTTGACGTCCCGAGGGACGTTATAG

3639 pst1,

cDNA clone
C2

GlnMetGluAspProThrPheLysGluAsnTyrArgPheHisAlaIleAsnGlyTyrIle
3661 CAGATGGAAGATCCCACTTTTAAAGAGAATTATCGCTTCCATGCAATCAATGGCTACATA
GTCTACCTTCTAGGGTGAAAATTTCTCTTAATAGCGAAGGTACGTTAGTTACCGATGTAT

3667 mboII, 3679 ahaIII,

MetAspThrLeuProGlyLeuValMetAlaGlnAspGlnArgIleArgTrpTyrLeuLeu
3721 ATGGATACACTACCTGGCTTAGTAATGGCTCAGGATCAAAGGATTCGATGGTATCTGCTC
TACCTATGTGATGGACCGAATCATTACCGAGTCCTAGTTTCCTAAGCTACCATAGACGAG

3753 binI,

SerMetGlySerAsnGluAsnIleHisSerIleHisPheSerGlyHisValPheThrVal
3781 AGCATGGGCAGCAATGAAAACATCCATTCTATTCATTTCAGTGGACATGTGTTCACTGTA
TCGTACCCGTCGTTACTTTTGTAGGTAAGATAAGTAAAGTCACCTGTACACAAGTGACAT

ArgLysLysGlu
3841 CGAAAAAAAGAG
GCTTTTTTTCTC

## Claims

1. A process for the synthesis of a polypeptide fragment of human Factor VIIIC, obtainable by thrombin degradation of Factor VIIIC,
which
(a) can be isolated from the thrombin degradation product of human Factor VIIIC by preparative SDS gel electrophoresis,
(b) has a molecular weight of 77/80 kd,
(c) has an amino acid composition of 608-634 amino acids consisting of:
| Amino acid | No. of Units | Amino acid | No. of Units |
|---|---|---|---|
| Asp | 54 - 58 | Ala | 33 - 35 |
| Glu | 74 - 76 | Pro | 30 - 33 |
| Cys | 12 - 14 | Tyr | 25 |
| Ser | 44 - 47 | Val | 44 - 46 |
| Gly | 46 - 51 | Met | 17 |
| His | 8 - 12 | Ile | 33 - 35 |
| Arg | 29 - 32 | Leu | 48 - 49 |
| Thr | 29 - 35 | Phe | 31 - 32 |
| | | Lys | 41 - 47 |
and
(d) can be convened by thrombin to a polypeptide of a molecular weight of 67/70 kD in which the amino acids numbered 2-22 from its amino terminus are: 
Phe-Gln-Lys-Lys-Thr-Arg-His-Tyr-Phe-Ile-Ala-Ala-Val-
-Glu-Arg-Leu-Trp-Asp-Tyr-Gly-Met,
 which comprises:
(A) Preparing a polynucleotide probe corresponding to at least one of the following amino acid sequences 
Phe-Gln-Lys-Lys-Thr-Arg-His-Tyr-Phe-Ile-Ala-Ala-
-Val-Glu-Arg-Leu-Trp-Asp-Tyr-Gly-Met,
Val-Thr-Gly-Val-Thr-Thr-Gln-Gly-Val-Lys
and
Met-Glu-Val-Leu-Gly-Cys-Glu-Ala-Gln-Asp-Leu-Tyr,
(B) adding the probe to a genomic DNA library and isolating a hybridizing DNA fragment,
(C) subcloning and sequencing the isolated genomic DNA fragment,
(D) probing a cDNA library with an appropriate part of the genomic DNA fragment or with a polynucleotide as defined above in A and isolating a hybridizing cDNA clone,
(E) subcloning and sequencing the isolated cDNA fragment,
(F) inserting the cDNA fragment into a suitable expression vector
and
(G) expressing the corresponding polypeptide fragment of human Factor VIIIC from a host compatible for that expression vector.

2. A DNA sequence of at least 200 bp having an open reading frame throughout said sequence and encoding a portion comprising 321 amino acids of the N terminal end of the 77/80 kd polypeptide fragment of human Factor VIIIC.

3. A DNA sequence according to claim 2, including the following DNA sequence:

glu ile thr arg thr thr leu gln
GAA ATA ACT CGT ACT ACT CTT CAG

ser asp gln glu glu ile asp tyr asp asp thr ile ser val glu
TCA GAT CAA GAG GAA ATT GAC TAT GAT GAT ACC ATA TCA GTT GAA

met lys lys glu asp phe asp ile tyr asp glu asp glu asn gln
ATG AAG AAG GAA GAT TTT GAC ATT TAT GAT GAG GAT GAA AAT CAG

ser pro arg ser phe gln lys lys thr arg his tyr phe ile ala
AGC CCC CGC AGC TTT CAA AAG AAA ACA CGA CAC TAT TTT ATT CGT

ala val glu arg leu trp asp tyr gly met ser ser ser pro his
GCA GTG GAG AGG CTC TGG GAT TAT GGG ATG AGT AGC TCC CCA CAT

val leu arg asn arg
GTT CTA AGA AAC AGG.

4. A DNA sequence according to claim 3, including the following DNA sequence:

N-termini 80/77 kD
GluIleThrArgThrThrLeuGlnSerAspGlnGluGluIleAspTyrAsp
GAAATAACTCGTACTACTCTTCAGTCAGATCAAGAGGAAATTGACTATGAT
CTTTATTGAGCATGATGAGAAGTCAGTCTAGTTCTCCTTTAACTGATACTA

AspThrIleSerValGluMetLysLysGluAspPheAspIleTyrAspGluAspGluAsn
GATACCATATCAGTTGAAATGAAGAAGGAAGATTTTGACATTTATGATGAGGATGAAAAT
CTATGGTATAGTCAACTTTACTTCTTCCTTCTAAAACTGTAAATACTACTCCTACTTTTA
N-termini 70/67 kD

GlnSerProArgSerPheGlnLysLysThrArgHisTyrPheIleAlaAlaValGluArg
CAGAGCCCCCGCAGCTTTCAAAAGAAAACACGACACTATTTTATTGCTGCAGTGGAGAGG
GTCTCGGGGGCGTCGAAAGTTTTCTTTTGTGCTGTGATAAAATAACGACGTCACCTCTCC

LeuTrpAspTyrGlyMetSerSerSerProHisValLeuArgAsnArgAlaGlnSerGly
CTCTGGGATTATGGGATGAGTAGCTCCCCACATGTTCTAAGAAACAGGGCTCAGAGTGGC
GAGACCCTAATACCCTACTCATCGAGGGGTGTACAAGATTCTTTGTCCCGAGTCTCACCG

SerValProGlnPheLysLysValValPheGlnGluPheThrAspGlySerPheThrGln
AGTGTCCCTCAGTTCAAGAAAGTTGTTTTCCAGGAATTTACTGATGGCTCCTTTACTCAG
TCACAGGGAGTCAAGTTCTTTCAACAAAAGGTCCTTAAATGACTACCGAGGAAATGAGTC

ProLeuTyrArgGlyGluLeuAsnGluHisLeuGlyLeuLeuGlyProTyrIleArgAla
CCCTTATACCGTGGAGAACTAAATGAACATTTGGGACTCCTGGGGCCATATATAAGAGCA
GGGAATATGGCACCTCTTGATTTACTTGTAAACCCTGAGGACCCCGGTATATATTCTCGT

GluValGluAspAsnIleMetValThrPheArgAsnGlnAlaSerArgProTyrSerPhe
GAAGTTGAAGATAATATCATGGTAACTTTCAGAAATCAGGCCTCTCGTCCCTATTCCTTC
CTTCAACTTCTATTATAGTACCATTGAAAGTCTTTAGTCCGGAGAGCAGGGATAAGGAAG

TyrSerSerLeuIleSerTyrGluGluAspGlnArgGlnGlyAlaGluProArgLysAsn
TATTCTAGCCTTATTTCTTATGAGGAAGATCAGAGGCAAGGAGCAGAACCTAGAAAAAAC
ATAAGATCGGAATAAAGAATACTCCTTCTAGTCTCCGTTCCTCGTCTTGGATCTTTTTTG

PheValLysProAsnGluThrLysThrTyrPheTrpLysValGlnHisHisMetAlaPro
TTTGTCAAGCCTAATGAAACCAAAACTTACTTTTGGAAAGTGCAACATCATATGGCACCC
AAACAGTTCGGATTACTTTGGTTTTGAATGAAAACCTTTCACGTTGTAGTATACCGTGGG

ThrLysAspGluPheAspCysLysAlaTrpAlaTyrPheSerAspValAspLeuGluLys
ACTAAAGATGAGTTTGACTGCAAAGCCTGGGCTTATTTCTCTGATGTTGACCTGGAAAAA
TGATTTCTACTCAAACTGACGTTTCGGACCCGAATAAAGAGACTACAACTGGACCTTTTT

AspValHisSerGlyLeuIleGlyProLeuLeuValCysHisThrAsnThrLeuAsnPro
GATGTGCACTCAGGCCTGATTGGACCCCTTCTGGTCTGCCACACTAACACACTGAACCCT
CTACACGTGAGTCCGGACTAACCTGGGGAAGACCAGACGGTGTGATTGTGTGACTTGGGA

AlaHisGlyArgGlnValThrValGlnGluPheAlaLeuPhePheThrIlePheAspGlu
GCTCATGGGAGACAAGTGACAGTACAGGAATTTGCTCTGTTTTTCACCATCTTTGATGAG
CGAGTACCCTCTGTTCACTGTCATGTCCTTAAACGAGACAAAAAGTGGTAGAAACTACTC

ThrLysSerTrpTyrPheThrGluAsnMetGluArgAsnCysArgAlaProCysAsnIle
ACCAAAAGCTGGTACTTCACTGAAAATATGGAAAGAAACTGCAGGGCTCCCTGCAATATC
TGGTTTTCGACCATGAAGTGACTTTTATACCTTTCTTTGACGTCCCGAGGGACGTTATAG

GlnMetGluAspProThrPheLysGluAsnTyrArgPheHisAlaIleAsnGlyTyrIle
CAGATGGAAGATCCCACTTTTAAAGAGAATTATCGCTTCCATGCAATCAATGGCTACATA
GTCTACCTTCTAGGGTGAAAATTTCTCTTAATAGCGAAGGTACGTTAGTTACCGATGTAT

MetAspThrLeuProGlyLeuValMetAlaGlnAspGlnArgIleArgTrpTyrLeuLeu
ATGGATACACTACCTGGCTTAGTAATGGCTCAGGATCAAAGGATTCGATGGTATCTGCTC
TACCTATGTGATGGACCGAATCATTACCGAGTCCTAGTTTCCTAAGCTACCATAGACGAG

SerMetGlySerAsnGluAsnIleHisSerIleHisPheSerGlyHisValPheThrVal
AGCATGGGCAGCAATGAAAACATCCATTCTATTCATTTCAGTGGACATGTGTTCACTGTA
TCGTACCCGTCGTTACTTTTGTAGGTAAGATAAGTAAAGTCACCTGTACACAAGTGACAT

ArgLysLysGlu
CGAAAAAAAGAG
GCTTTTTTTCTC

5. An extrachromosomal element comprising a replication system compatible with a host cell capable of growth in culture and a DNA sequence according to one of claims 2 to 4.

6. An extrachromosomal element according to claim 5, including transcriptional and translational regulatory signals compatible with said host in juxtaposition to said DNA sequence for expression of said DNA sequence.

7. An extrachromosomal element according to claim 5 or 6, wherein said host cell is a unicellular microorganism.

8. An extrachromosomal element according to claim 5 or 6, wherein said host is a mammalian cell.

9. An extrachromosomal element according to claim 8, wherein said mammalian cell is a COS cell.

10. A complex probe having the following formula:

TCT GTA ATG AAA TAG CGA CGA CAC CTT TCT GAC ACC CTA ATG CCG TAC -
  C   G       G       G   G       C   C          G

11. A DNA sequence of about 10 to 25 kbp derived from the human genome encoding at least a portion of human Factor VIIIC and hybridizing with a probe according to claim 10.

12. A DNA construct comprising a DNA sequence according to claim 11 joined to a unicellular microorganism cloning vector.
